# EUROPEAN PATENT SPECIFICATION

(11) **EP 1 271 240 B1**
(45) Date of publication and mention of the grant of the patent: **28.06.2006**
(21) Application number: 02254138.7
(22) Date of filing: 13.06.2002
(51) Int. Cl.: G03C 7/38, G03C 7/32, C07D 487/04

(54) **Silver halide color photographic material**
Farbphotographisches Silberhalogenidmaterial
Matériau photographique couleur à l'halogénure d'argent

(30) Priority: 18.06.2001 JP 2001183165
(43) Date of publication of application: 02.01.2003
(73) Proprietor: KONICA CORPORATION, Tokyo (JP)
(72) Inventor: Ikesu, Saturo, Hino-shi, Tokyo 191-8511 (JP); Nogi, Kyoko, Hino-shi, Tokyo 191-8511 (JP); Suzuki, Takatugu, Hino-shi, Tokyo 191-8511 (JP); Chen, Zheliu, Hino-shi, Tokyo 191-8511 (JP)
(74) Representative: Ellis-Jones, Patrick George Armine

(56) References cited:
- EP-A- 0 802 454
- PATENT ABSTRACTS OF JAPAN vol. 2002, no. 05, 3 May 2002 (2002-05-03) & JP 2002 014445 A (KONICA CORP), 18 January 2002 (2002-01-18) -& JP 2002 014445 A (KONICA CORP) 18 January 2002 (2002-01-18)
- PATENT ABSTRACTS OF JAPAN vol. 0161, no. 93 (P-1349), 11 May 1992 (1992-05-11) & JP 4 027947 A (KONICA CORP), 30 January 1992 (1992-01-30) -& JP 04 027947 A (KONICA CORP) 30 January 1992 (1992-01-30)

## Description

### FIELD OF THE INVENTION

The present invention relates to a silver halide color photographic material comprising a coupler containing a specific ballast group.

### BACKGROUND OF THE INVENTION

In a silver halide color photographic material (hereinafter, also denoted simply as color photographic material or photographic material), a technique is useful to incorporate a coupler into a photographic emulsion layer, in which a hydrophobic ballast group is introduced into a coupler molecule and after dissolved in an organic solvent, the coupler is incorporated in the form of an emulsion dispersed in a hydrophilic colloid such as gelatin.

Basic properties required for couplers include enhanced solubility in high boiling organic solvents, superior dispersibility and dispersion stability in a silver halide emulsion, without easily forming precipitates, exhibiting superior spectral absorption characteristics and color tone and forming clear dye images over a wide color reproduction region, and a method of preparing the coupler from low-priced raw material in a simple and reproducible manner and in a relatively high yield.

Ballast groups contained in couplers play a great role, affecting the foregoing performances and specific ballast groups are proposed in JP-B Nos. 44-3660, 48-25655, 48-25932, 48-25934, 49-16057 and 51-40804 (hereinafter, the term, JP-B means a published Japanese Patent); JP-A Nos. 47-4481, 49-8228, 50-19435, 51-126831, 52-86333, 56-30126, 57-146251, 58-42045, 59-177557, 60-24547, and 9-281672 (hereinafter, the term JP-A means an unexamined, published Japanese Patent Application); U.S. Patent Nos. 2,908,573, 2,920,961, and 3,227,544.

However, these ballast groups were still insufficient for satisfying the foregoing performances. Specifically in pyrazolotriazole type cyan couplers, a further improvement is desired in terms of solubility in high boiling organic solvents and superior color reproduction in the relatively high density region, and to obtain the coupler from low-priced raw material in a simple and reproducible manner and in a relatively high yield.

### SUMMARY OF THE INVENTION

In view of the foregoing problems, it is an object of the invention to provide a silver halide color photographic material comprising a coupler obtained in the preparation method for a coupler from low-priced raw material in a simple and reproducible manner and in a relatively high yield.

It is a second object of the invention to provide a color photographic material comprising a coupler exhibiting superior solubility in organic solvents (including high-boiling and low-boiling organic solvents).

It is a third object of the invention to provide a color photographic material exhibiting superior lasting quality with respect to color reproduction of formed dye images.

The foregoing objects of the invention can be accomplished by the following constitution:
1. A silver halide color photographic material comprising a support having thereon at least one silver halide emulsion layer comprising a coupler represented by formula (2), (3) or (4) : 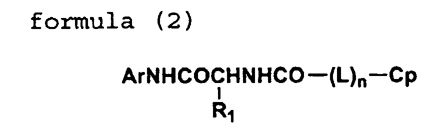 wherein Ar is an aryl group or a heterocyclic group; R₁ is an alkyl group, an aryl group or a heterocyclic group; L is a bivalent linkage group; n is 0 or 1; and Cp is a pyrazolotriazole coupler residue; 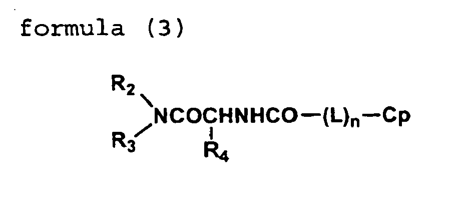 wherein R₂, R₃, and R₄ are each an alkyl group, an aryl group or a heterocyclic group, or R₂ and R₃ combine with each other to form a ring; L is a bivalent linkage group; n is 0 or 1; and Cp is a pyrazolotriazole coupler residue; 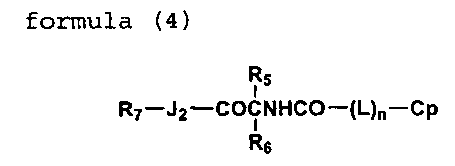 wherein R₅ is an unsubstituted alkyl group having 5 or more carbon atoms; R₆ is a hydrogen atom, an alkyl group, an aryl group or a heterocyclic group; R₇ is an alkyl group, an aryl group or a heterocyclic group; J₂ is -O- or -NR₁₁-, in which R₁₁ is a hydrogen atom, an alkyl group, an aryl group or a heterocyclic group; L is a bivalent linkage group; n is 0 or 1; Cp is a pyrazolotriazole coupler residue.
2. The photographic material described above, wherein the coupler residue of formulas (2), (3) and (4) is represented by the following formula (6) : 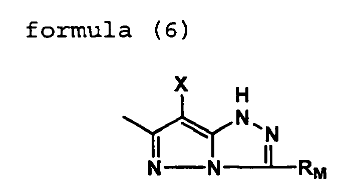
wherein X is a hydrogen atom, a halogen atom or a group capable of being released upon coupling with an oxidation product of a color developing agent; and R_{M} is a univalent substituent group.

### BRIEF EXPLANATION OF THE DRAWING

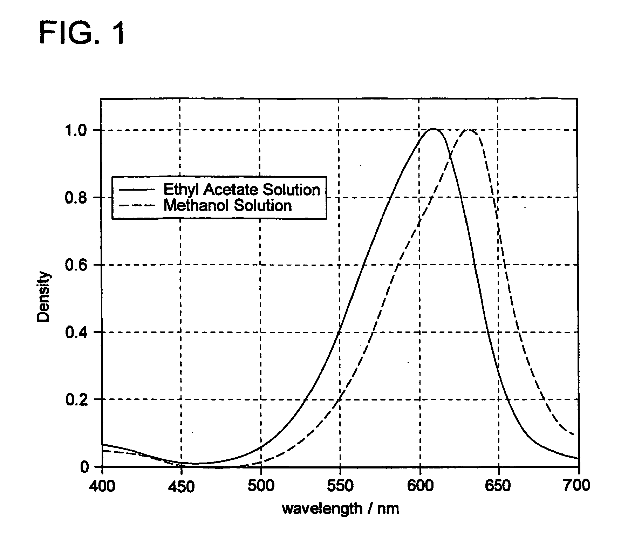

Fig. 1 shows absorption spectrum of exemplified compound 8-9.

### DETAILED DESCRIPTION OF THE INVENTION

Cp in formula (2), (3) and (4) is a pyrazolotriazole coupler residue and in the coupler residue, representative examples of a yellow coupler residue include those described in U.S. Patent Nos. 2,298,443, 2,407,210, 2,875,057, 3,048,194 and 3,447,928; and Farbkuppler eine Literatur ubersicht Agfa Mitteilung (Band II), 112-126 (1961).

Examples of a magenta coupler residue include those described in U.S. Patent Nos. 2,369, 489, 2,343,708, 2,311,082, 2,600,788, 2,908,573, 3,062,653, 3,152,896, 3,519,429, 3,725,067, 4,540,654; JP-A No. 59-162648 and also in Farbkuppler eine Literaturübersicht Agfa Mitteilung (Band II), 126-156 (1961).

Examples of a cyan coupler residue include those described in U.S. Patent Nos. 2,367,531, 2,423,730, 2,772,162, 2,895,826, 3,002,836, 3,034,892, 3,041,236; JP-A No. 64-554; and also in Farbkuppler eine Literaturübersicht Agfa Mitteilung (Band II), 156-175 (1961).

The coupler residue represented by the foregoing formula (6) is specifically preferred.

In formula (6), X represents a hydrogen atom, halogen atom or a group capable of being released upon reaction with an oxidation product of a color developing agent (i.e., a coupling-off group). Examples of an atom or group capable of being released upon reaction with an oxidation product of a color developing agent include a hydrogen atom, halogen atom (e.g., chlorine, bromine, iodine), alkyleneoxy, alkoxy, aryloxy, heterocyclic-oxy, acyloxy, sulfonyloxy, alkoxycarbonyloxy, aryloxycarbonylalkyloxazolyloxy, alkoxyoxazolyloxy, alkylthio, arylthio, heterocyclic-thio, alkyloxythiocarbonylthio, acylamino, sulfonamido, N-bonded nitrogen containing heterocycle, alkyloxycarbonylamino, aryloxycarbonylamino, and carboxyl. Of these, a hydrogen atom, halogen atom alkoxy group, and aryloxy group are preferred.

In formula (6), R_{M} represents a univalent substituent group. The univalent group represented by R_{M} is not specifically limited and representative examples thereof include alkyl, aryl, anilino, acylamino, sulfonamido, alkylthio, arylthio, alkenyl, and cycloalkyl. In addition thereto, a halogen atom, cycloalkenyl, alkynyl, heterocyclic group, sulfinyl, phosphonyl, acyl, carbamoyl, sulfamoyl, cyano, alkoxy, aryloxy, heterocyclic-oxy, siloxy, acyloxy, sulfonyloxy, carbamoyloxy, amino, alkylamino, imido, ureido, sulfamoylamino, alkoxycarbonylamino, aryloxycarbonylamino, alkoxycarbonyl, aryloxycarbonyl, heterocyclic-thio, thioureido, carboxy, hydroxy, mercapto, nitro, and sulfo groups, spiro-compound residue and bridged hydrocarbon residue are also included.

The alkyl group represented by R_{M} is preferably one having 1 to 32 carbon atoms, which may be straight chain or branched. The aryl group represented by R_{M} is preferably phenyl. Acyl amino group include alkylcarbonylamino and arylcarbonylamino. The sulfonamido represented by R_{M} include, for example, alkylsulfonylamino and arylsulfonylamino. The alkyl component and aryl component in the alkylthio group and arylthio group include an alkyl groups and aryl groups represented by the foregoing R_{M}. The alkenyl group represented by R_{M} contains preferably 2 to 32 carbon atoms, the cycloalkyl group contains preferably 3 to 12 carbon atoms, and more preferably 5 to 7 carbon atoms. The alkenyl group may be straight chain or branched. The cycloalkenyl group represented by R_{M} contains preferably 3 to 12 carbon atoms, and more preferably 5 to 7 carbon atoms. The sulfonyl group include an alkylsulfonyl and arylsulfonyl. The sulfinyl group represented by R_{M} include alkylsulfinyl and aryl sulfinyl; the phosphonyl group represented by R_{M} alkylphosphonyl, alkoxyphosphonyl, aryloxyphosphonyl and arylphosphonyl; the acyl group represented by R_{M} include alkylcarbonyl, and arylcarbonyl; the carbamoyl group represented by R_{M} include alkylcarbamoyl and arylcarbamoyl; the sulfamoyl group represented by R_{M} include alkylsulfamoyl and arylsulfamoyl; the acyloxy group represented by R_{M} include alkycarbonyloxy and arylcarbonyloxy; the sulfonyloxy group include alkylsulfonyloxy and arysulfonyloxy; the carbamoyloxy represented by R_{M} include alkylcarbamoyloxy and arylcarbamoyloxy; the ureido group represented by R_{M} include alklylureido and arylureido; the sulfamoylamino group include alkylsulfamoylamino and arylsulfamoylamino; the heterocyclic group represented by R_{M} is preferably a 5- to 7-membered ring, including, for example, 2-furyl, 2-thienyl, 2-pyridinyl, 2-benzothiazolyl, 1-pyrrolyl and 1-tetrazolyl; the heterocyclic-oxy group represented by R_{M} is preferably a 5-to 7-membered ring, including, for example, 3,4,5,6-tetrahydropyranyl-2-oxy, and 1-phenyltetrazole-5-oxy; the heterocyclic-thio group represented by R_{M} is preferably a 5-to 7-membered ring, including, for example, 2-pyridylthio, 2-benzothiazolylthio, 2,4-diphenoxy-1,3,5-triazole-6-thio; the siloxy group represented by R_{M} includes trimethylsiloxy, triethylsiloxy and dimethylbutylsiloxy; the imido group represented by R_{M} includes succinic acid imido (or succinimido), 3-heptadecylsuccinic acid imido, phthalic acid imido (or phthalimide) and glutaric acid imido; the spio-compound residue represented by R_{M} is, for example, spiro[3,3]heptane-1-yl; the bridged hydrocarbon compound residue is, for example, bicyclo[2,2,1]heptane-1-yl, tricyclo[3,3,1,1,³⁷]decane-1-yl and 7,7-dimethyl-bicyclo[2,21]heptane-1-yl. The substituent group represented by R_{M} is preferably alkyl or aryl, and more preferably aryl.

Next, the couplers represented by the foregoing formula (2), (3) or (4) will be described.

In formula (2), Ar represents an aryl group or heterocyclic group. The aryl groups represented by Ar include, for example, phenyl and naphthyl. The heterocyclic group represented by Ar is preferably a 5- to 7-membered ring, including, for example, 2-furyl, 2-thienyl, 2-pyridinyl, 2-benzothiazolyl, 1-pyrrolyl and 1-tetrazolyl. These aryl or heterocyclic group represented by Ar may be substituted. Substituent groups are not specifically limited and specific examples thereof include an alkyl, aryl, anilino, acylamino, sulfonamido, alkylthio, arylthio, and cycloalkyl groups. Other examples include a halogen atom, cycloalkenyl, alkynyl, heterocyclic group, sulfinyl, phosphonyl, acyl, carbamoyl, sulfamoyl, cyano, alkoxy, aryloxy, heterocyclic-oxy, siloxy, acyloxy, sulfonyloxy, carbamoyloxy, amino, alkylamino, imido, ureido, sulfamoylamino, alkoxycarbonylamino, aryloxycarbonylamino, alkoxcarbonyl, aryloxycarbonyl, heterocyclic-thio, thioureido, carboxy, hydroxy, mercapto, nitro, and sulfo groups, spiro-compound residue and bridged hydrocarbon residue.

In formula (2), R₁ is preferably an alkyl or aryl group, more preferably alkyl group, and still more preferably an unsubstituted alkyl group. L represents a bivalent linkage group, and preferably an alkylene or arylene group; n is 0 or 1, and preferably 0. The coupler residue represented by Cp is a pyrazolotriazole, preferably a coupler residue represented by formula (6).

Next, the coupler represented by formula (3) will be described. In formula (3), R₂, R₃ and R₄ are each an alkyl group, aryl group or heterocyclic group, provided that R₂ and R₃ may combine with each other to form a ring; and examples thereof include an alkyl group such as methyl, ethyl, isopropyl, butyl or dodecyl; an aryl group such as phenyl; and a heterocyclic group, preferably 5- to 7- membered one, such as 2-furyl, 2-thienyl, 2-pyridyl, 2-benzothiazol, 1-pyrrolyl, or 1-tetrazolyl. R₂, R₃ and R₄ are preferably an alkyl group or aryl group. R₄ is preferably an alkyl or aryl group, more preferably an alkyl group, and still more preferably an unsubstituted alkyl group. L is a bivalent linkage group, and preferably an alkylene group or arylene group; n is 0 or 1, and preferably 0. The coupler residue represented by Cp is a pyrazolotriazole, preferably a coupler residue represented by formula (6).

Next, the coupler represented by formula (4) will be described. In formula (4), R₅ is an unsubstituted alkyl group having 5 or more carbon atoms, which may be straight chain or branched, including, for example, hexyl, octyl, decyl, dodecyl and 1,1,3-trimethylbutyl. R₆ is a hydrogen atom, an alkyl group, aryl group or heterocyclic group and examples of the alkyl, aryl and heterocyclic group and represented by R₆ include the same as cited in R₂, R₃ and R₄ in formula (3); R₆ is preferably a hydrogen atom or alkyl group, and more preferably a hydrogen atom. R₇ is preferably an alkyl group or aryl group. J₂ is preferably -NR₁₁-, in which R¹¹ is a hydrogen atom, an alkyl group, an aryl group or a heterocyclic group. Preferred examples of R₁₁ are those cited in R₂, R₃ and R₄ in formula (3). L is a bivalent linkage group, and preferably an alkylene group or arylene group; n is 0 or 1, and preferably 0. The coupler residue represented by Cp is a pyrazolotrizole, preferably the coupler residue represented by the foregoing formula (6).

Specific examples of the coupler represented by compounds (1-19) and (1-20) and formulae (2) to (4) are shown below, but the present invention is not limited to materials containing these couplers.

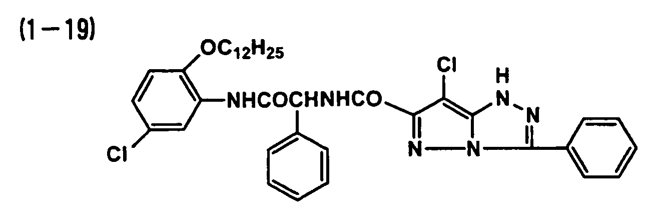

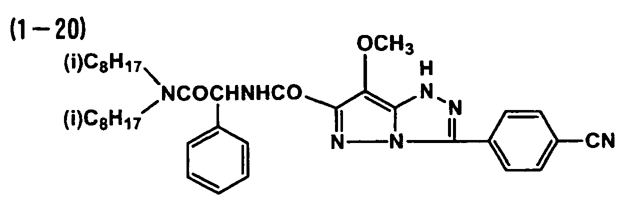

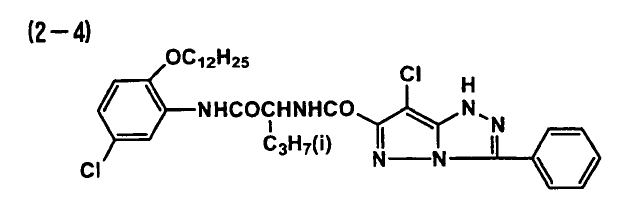

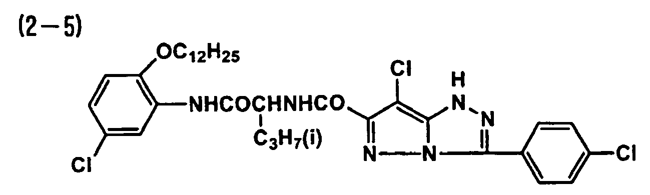

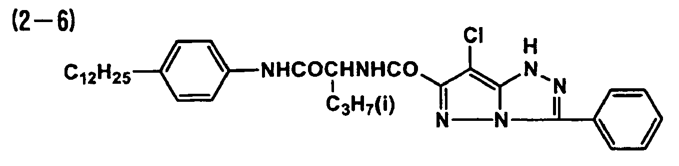

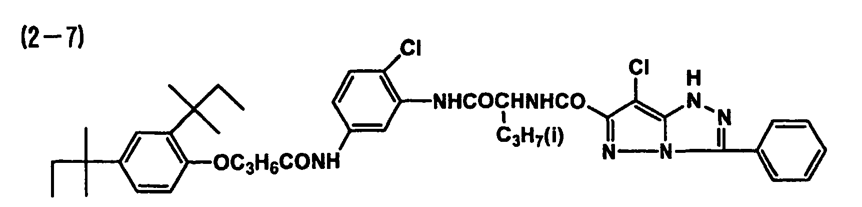

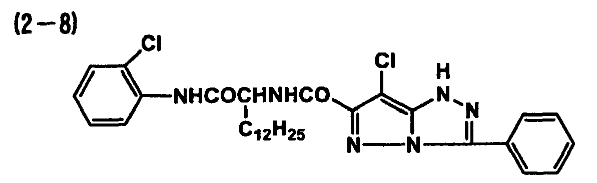

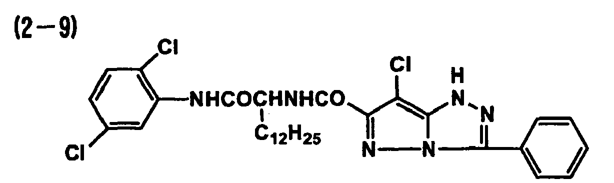

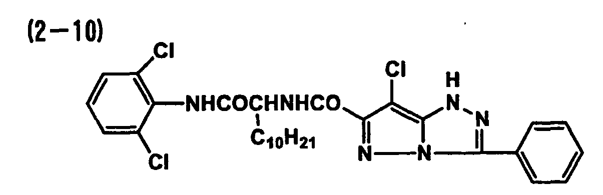

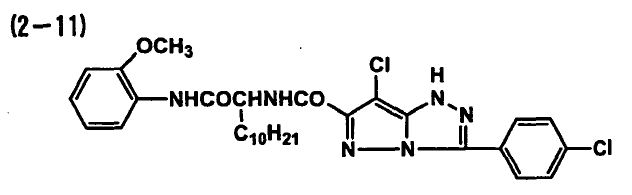

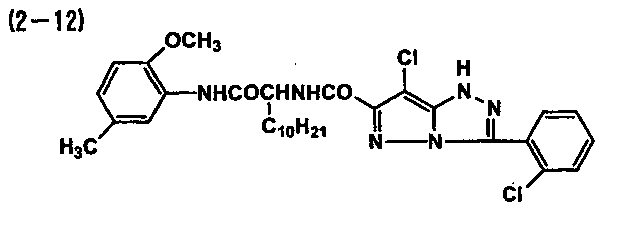

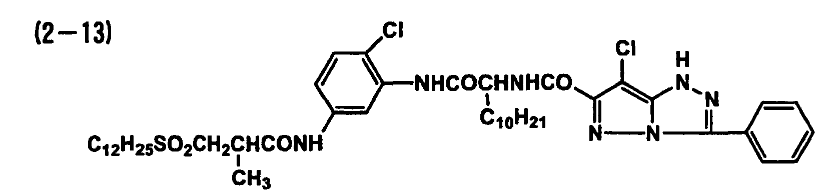

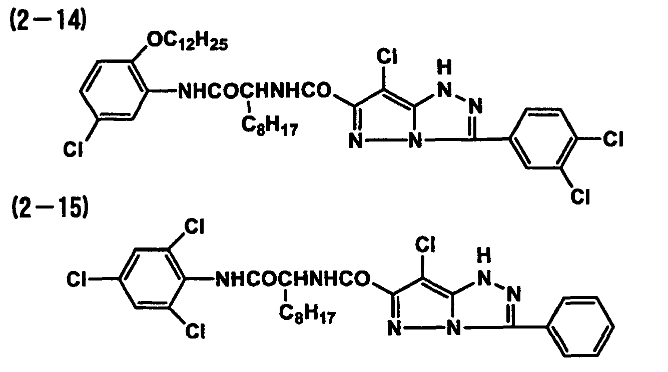

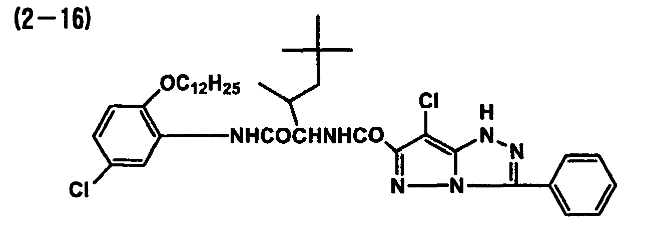

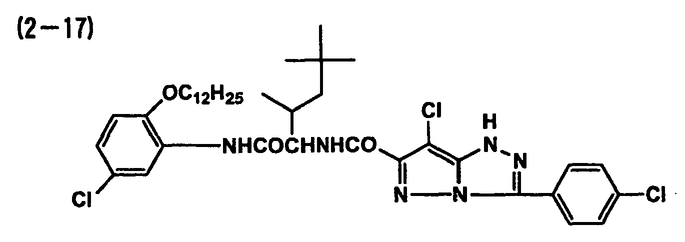

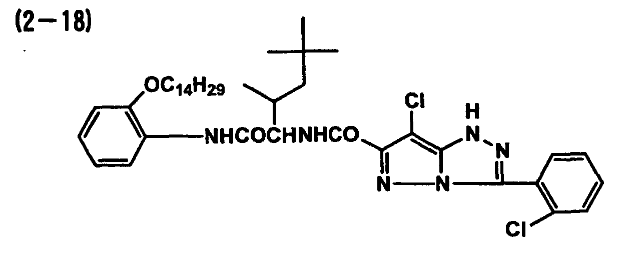

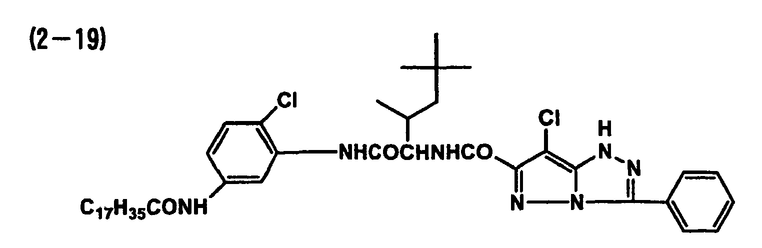

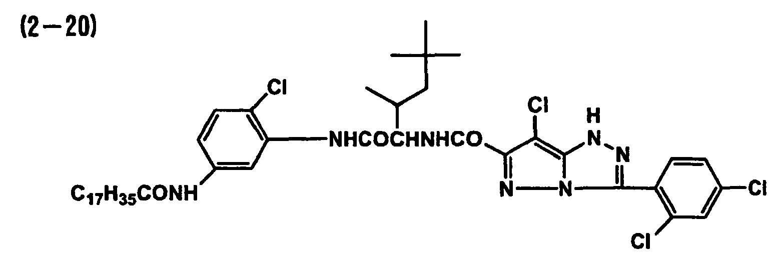

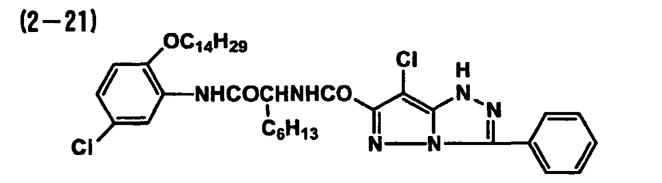

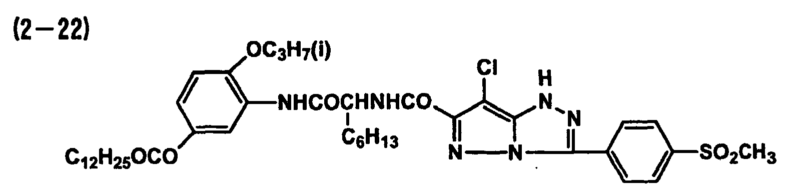

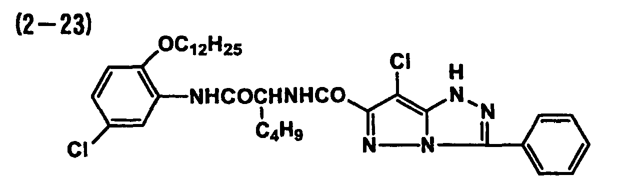

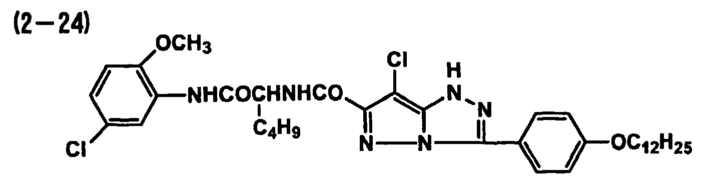

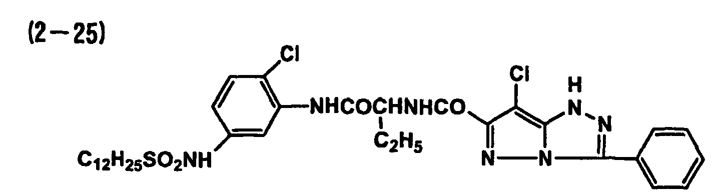

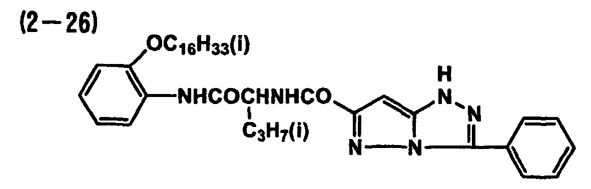

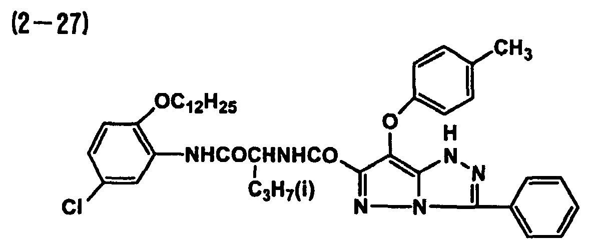

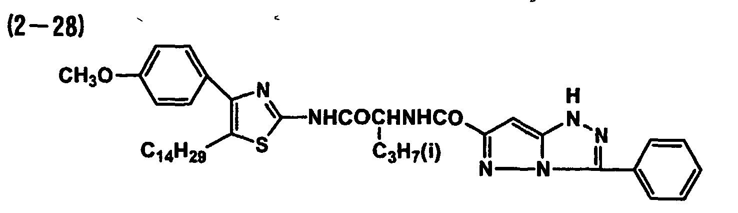

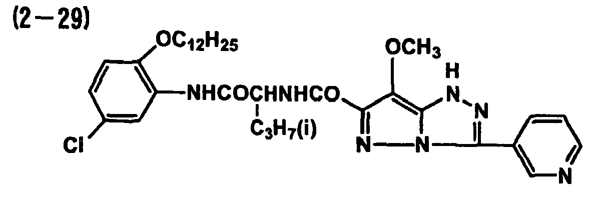

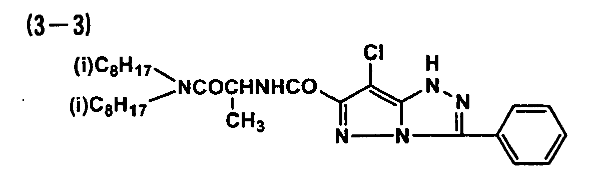

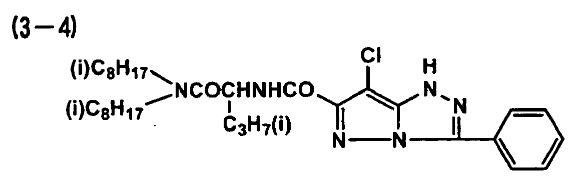

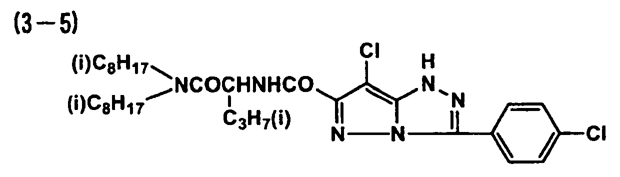

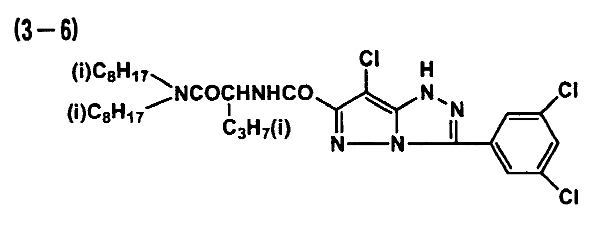

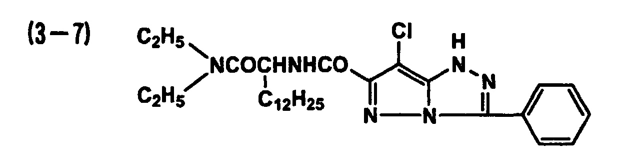

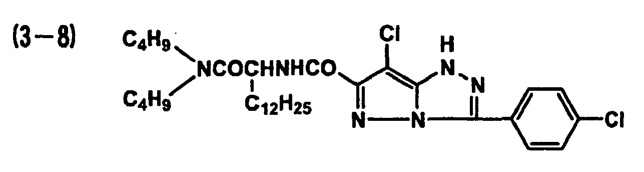

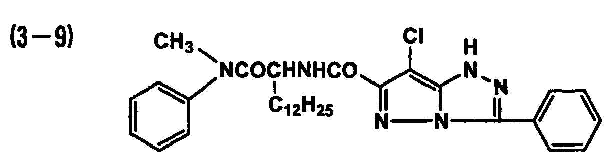

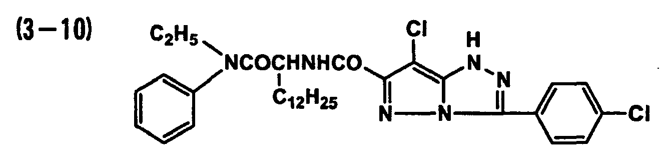

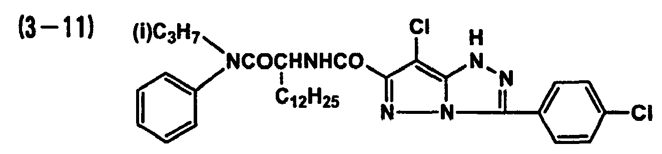

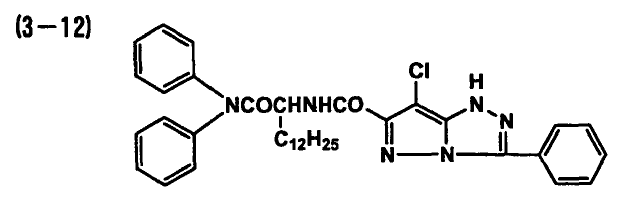

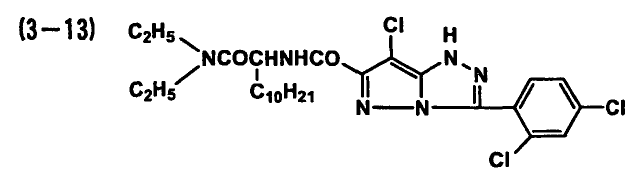

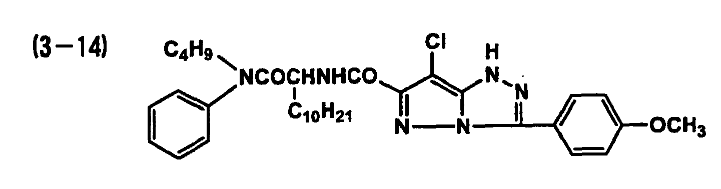

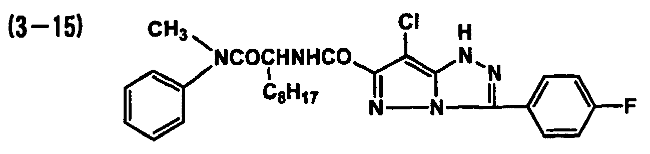

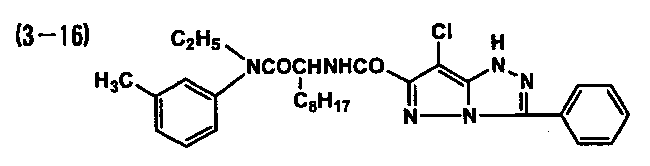

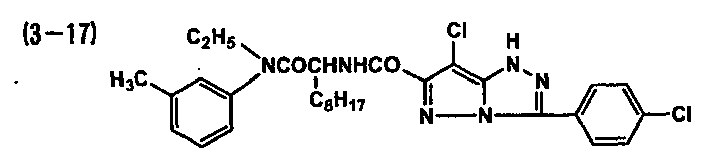

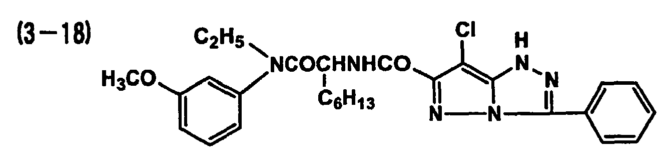

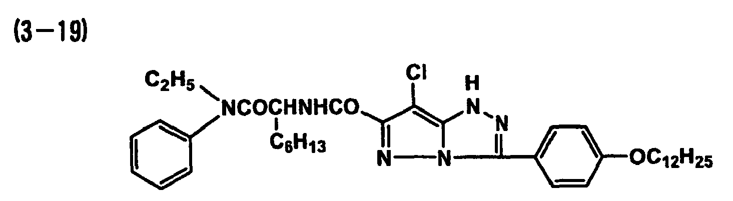

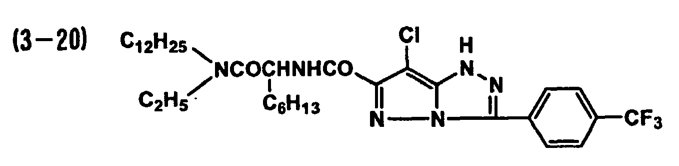

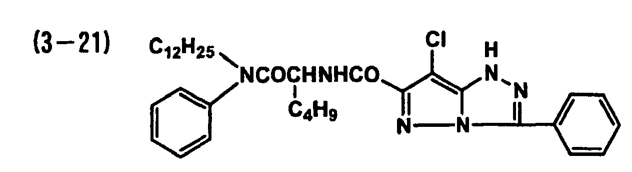

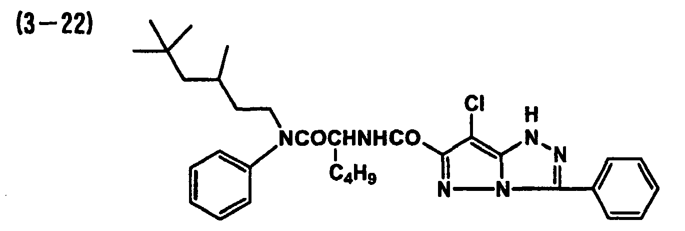

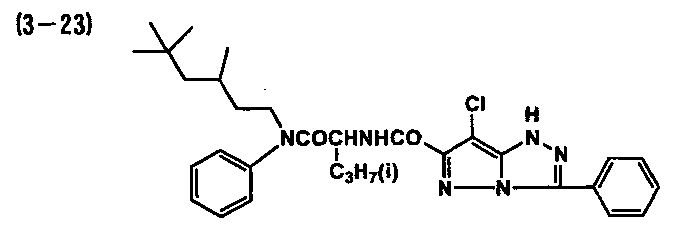

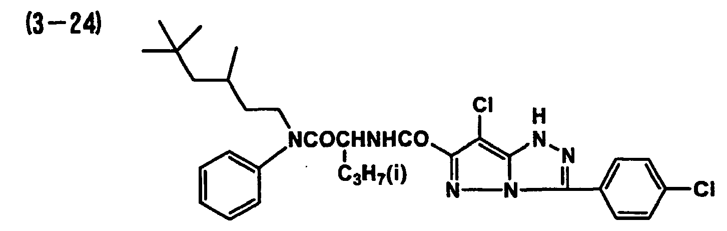

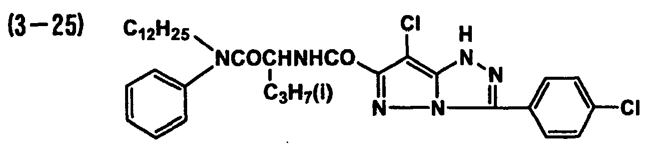

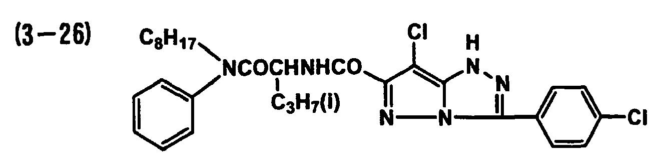

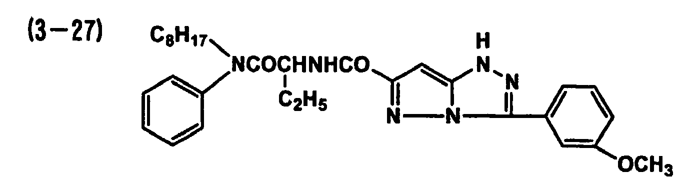

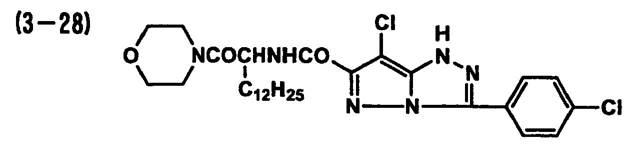

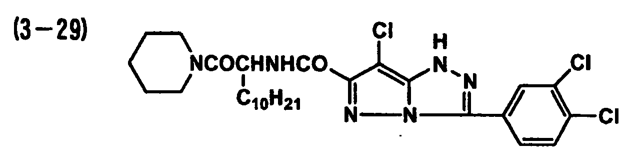

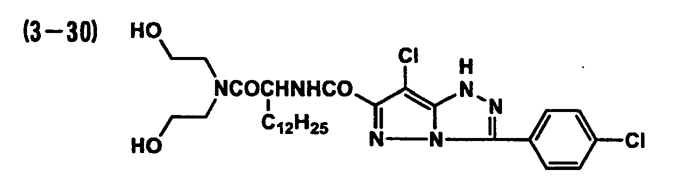

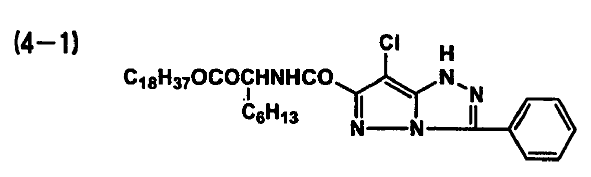

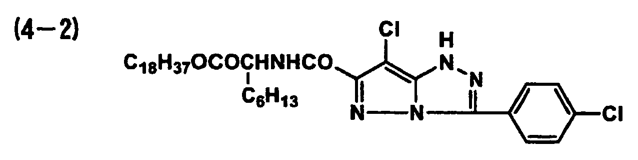

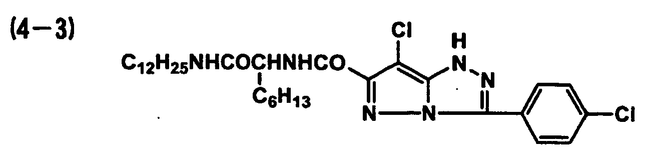

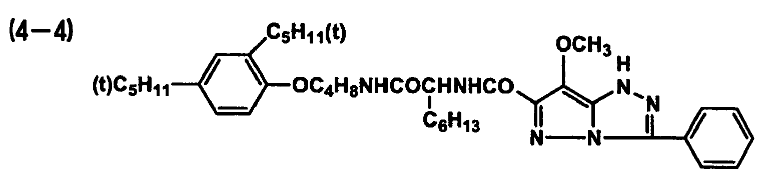

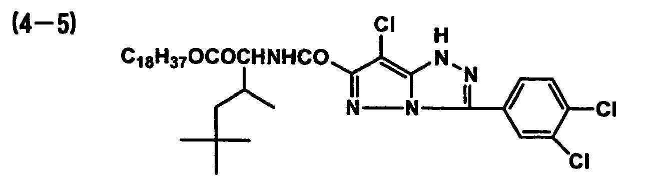

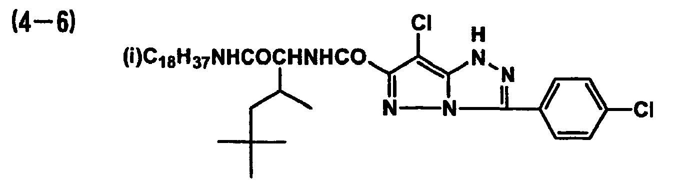

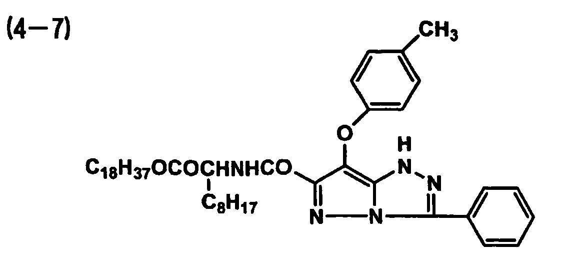

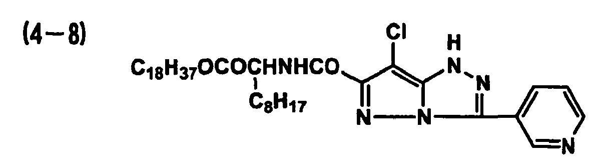

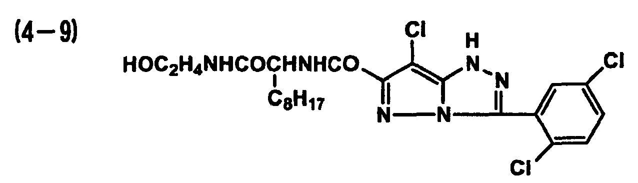

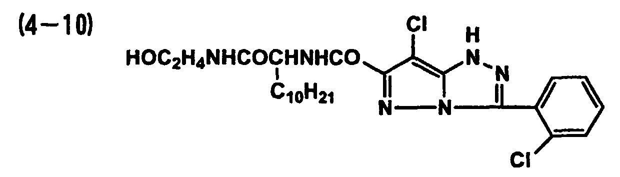

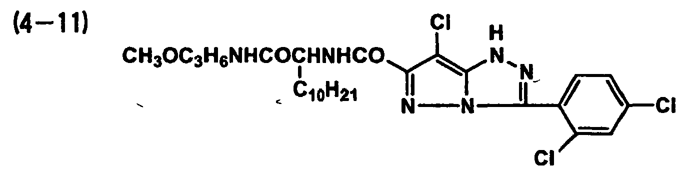

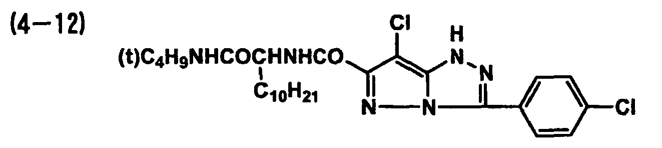

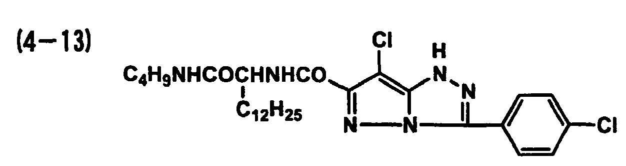

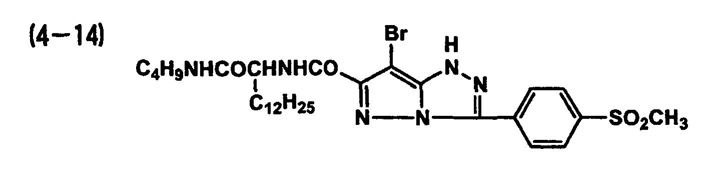

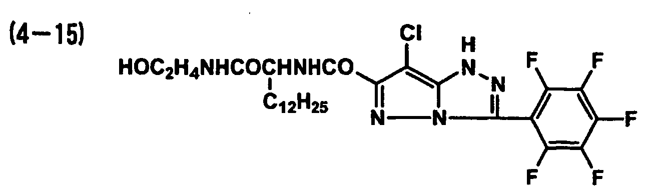

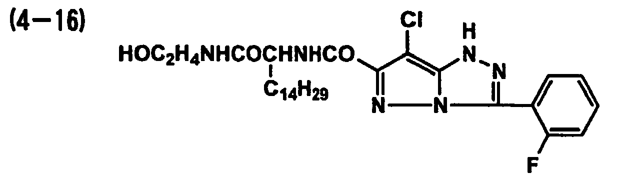

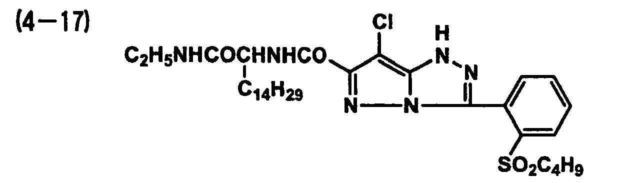

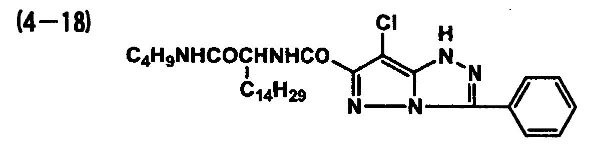

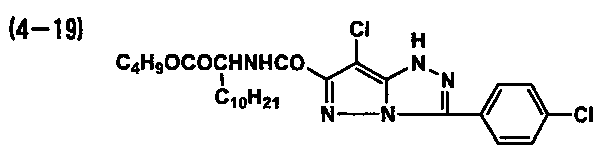

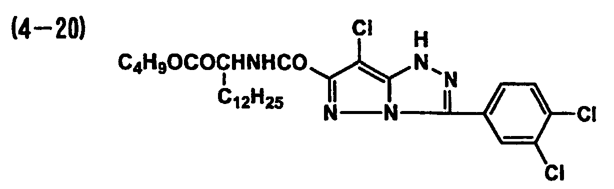

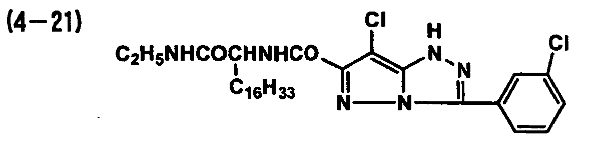

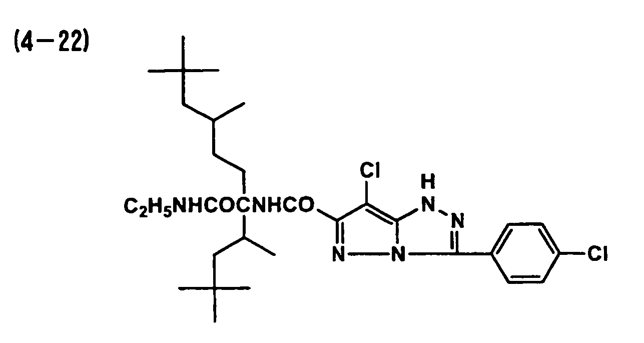

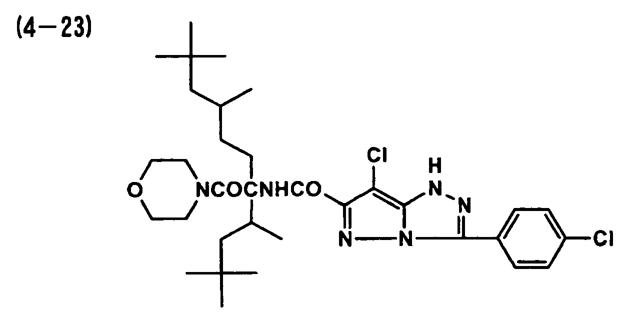

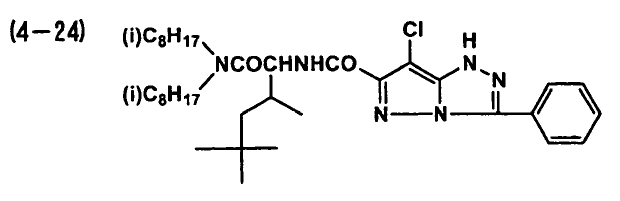

The couplers used in the invention can be synthesized in accordance with methods described in JP-A Nos. 8-171185, 8-311360, 8-339060 and 9-281672.

Dye formed from the foregoing couplers represented by formulas (2), (3) and (4) preferably are those which are represented by the following formulas (7), (8) and (9), respectively: 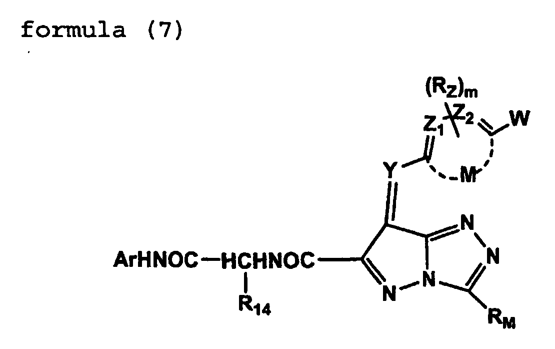 wherein W is -NRₓR_{y}, -OH or -OZ, in which Rₓ and R_{y} are each an alkyl group or an aryl group; Z is an alkali metal ion or a quaternary ammonium ion; R_{z} is a hydrogen atom, a halogen atom or a univalent substituent group; m is an integer of 1 to 3; Z₁ and Z₂ are each a nitrogen atom or C(R₂₇), in which R₂₇ is a univalent substituent group; M is an atomic group necessary to a 5- or 6-membered aromatic carbon ring or aromatic heterocyclic ring, together with Z₁, Z₂ and adjacent carbon atoms; Y is a nitrogen atom or CR₂₈, in which R₂₈ is a hydrogen atom, an alkyl group or an aryl group; R_{M} is a univalent substituent group; Ar is the same as Ar defined in formula (2) ; and R₁₄ is the same as R₁ defined in formula (2) ; 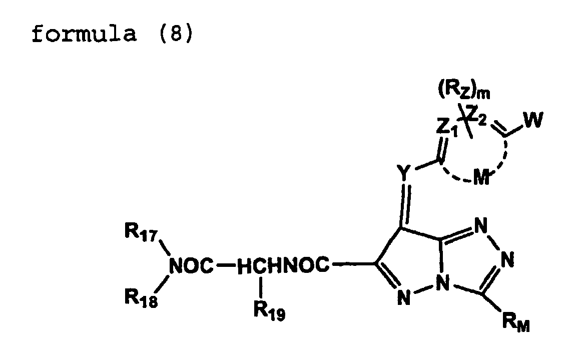 wherein W is -NRₓR_{y}, -OH or -OZ, in which Rₓ and R_{y} are each an alkyl group or an aryl group; Z is an alkali metal ion or a quaternary ammonium ion; R_{z} is a hydrogen atom, a halogen atom or a univalent substituent group; m is an integer of 1 to 3; Z₁ and Z₂ are each a nitrogen atom or C(R₂₇), in which R₂₇ is a univalent substituent group; M is an atomic group necessary to a 5- or 6-membered aromatic carbon ring or aromatic heterocyclic ring, together with Z₁, Z₂ and adjacent carbon atoms; Y is a nitrogen atom or CR₂₈, in which R₂₈ is a hydrogen atom, an alkyl group or an aryl group; R_{M} is a univalent substituent group; R₁₇, R₁₈ and R₁₉ are the same as R₂, R₃ and R₄, respectively, as defined in formula (3) ; 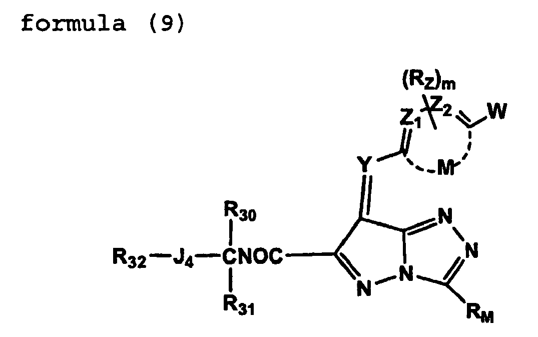 wherein W is -NRₓR_{y}, -OH or -OZ, in which Rₓ and R_{y} are each an alkyl group or an aryl group; Z is an alkali metal ion or a quaternary ammonium ion; R_{z} is a hydrogen atom, a halogen atom or a univalent substituent group; m is an integer of 1 to 3; Z₁ and Z₂ are each a nitrogen atom or C(R₂₇), in which R₂₇ is a univalent substituent group; M is an atomic group necessary to a 5- or 6-membered aromatic carbon ring or aromatic heterocyclic ring, together with Z₁, Z₂ and adjacent carbon atoms; Y is a nitrogen atom or CR₂₈, in which R₂₈ is a hydrogen atom, an alkyl group or an aryl group; R_{M} is a univalent substituent group; R₃₀, R₃₁, R₃₂ and J₄ are the same as R₅, R₆, R₇ and J₂, respectively, as defined in formula (4).

Next, the dyes represented by formulas (7) through (9) will be described further in detail.

In formulas (7) through (9), W represents -NRₓR_{y}, -OH or -OZ, in which Rₓ and R_{y} each represent an alkyl group or an aryl group. The alkyl group is preferably methyl, ethyl propyl or butyl, which may be substituted by a substituent group such as hydroxy or a sulfonamido group. The aryl group is preferably phenyl. Z represents an alkali metal ion or quaternary ammonium ion.

In the formulas, R_{z} represents a halogen atom (e.g., chlorine, bromine, fluorine), or a univalent substituent group. Examples of the substituent group include an alkyl group (e.g., methyl ethyl, isopropyl, hydroxyethyl, methoxymethyl, trifluoromethyl, t-butyl), cycloalkyl group (e.g., cyclopentyl, cyclohexyl), aralkyl group (e.g., benzyl, 2-phenethyl), aryl group (e.g., phenyl, naphthyl, p-tolyl, p-chlorophenyl), alkoxy group (e.g., methoxy, ethoxy, isopropoxy, n-butoxy), aryloxy group (e.g., phenoxy), cyano, acylamino group (e.g., acetylamino, propionylamino), alkylthio group (e.g., methylthio, ethylthio, n-butylthio), arylthio group (e.g., phenylthio), sulfonylamino group (e.g., methanesulfonylamino, benzenesulfonylamino), ureido group (e.g., 3-methylureido, 3,3-dimethylureido, 1,3-dimethylureido), sulfamoylamino group (e.g., dimethylsulfamoylamino), carbamoyl group (e.g., methylcarbamoyl, ethylcarbamoyl, dimethylcarbamoyl), sulfamoyl group (e.g., ethylsulfamoyl, dimethylsulfamoyl, ), alkoxycarbonyl group (e.g., methoxycarbonyl, ethoxycarbonyl), aryloxycarbonyl group (e.g., phenylcarbonyl), sulfonyl group (e.g., methanesulfonyl, butanesulfonyl, phenylsulfinyl), acyl group (e.g., acetyl, propanoyl, butyloyl), amino group (e.g., methylamino, ethylamino, dimethylamino), hydroxy group, nitro group, imido group (e.g., phthalimido), and heterocyclic group (e.g., pyridyl, benzimidazolyl, benzthiazolyl, benzoxazolyl).

The alkali metal ion represented by Z includes for example, sodium ion and potassium ion. The quaternary ammonium ion represented by Z includes an ammonium ion having 8 or more carbon atoms, such as trimethylbenzylammonium ion, tetrabutylammonium ion, and tetradecylammonium ion.

M represents an atomic group necessary to form an aromatic carbon ring or aromatic heterocyclic group, together with Z₁ and Z2 and carbon atoms adjacent thereto. Examples of the aromatic carbon ring or aromatic heterocyclic ring composed of M, Z₁, Z₂ and carbon atoms adjacent thereto include a benzene ring, pyridine ring, pyrimidine ring, pyridazine ring, pyrazine ring, triazine ring, tetrazine ring, pyrrole ring, furan ring, thiophene ring, thiazole ring, oxazole ring, imidazole ring, thiadiazole ring, and oxadiazole ring. Of these, a benzene ring and pyridine ring are preferred. Z₁ and Z₂ represent a nitrogen atom or C(R₂₇), in which R₂₇ is a univalent substituent group. The univalent substituent group is the same as cited in R_{z} described above; n is an integer of 1 to 3.

Y represents a nitrogen atom or CR₂₈, in which R₂₈ is a hydrogen atom, alkyl group or aryl group. Ar is the same Ar as defined in formula (2) ; R₁₄ is the same as R₁₄ as defined in formula (2) ; R₁₇, R₁₈ and R₁₉ are the same as R₂, R₃ and R₄, respectively, as defined in formula (3) ; R₃₀, R₃₁, R₃₂ and J₄ are the same as R₅, R₆, R₇ and J₂, as defined in formula (4). The univalent substituent group represented by R_{M} in formulas (7) to (9) is the same as R_{M} as defined in formula (6) and the preferred substituent group is the same as cited therein.

Specific examples of the dyes represented by formulas (7) to (9) are shown below but are not limited to these.

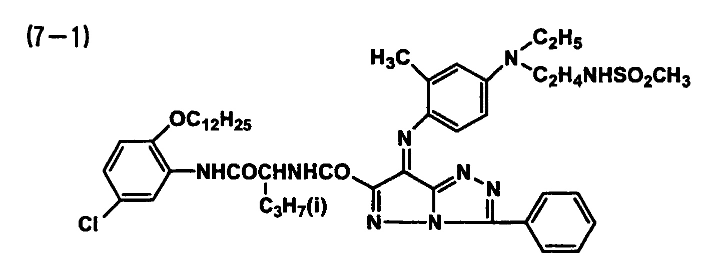

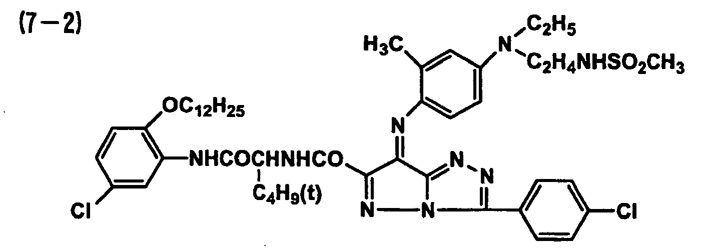

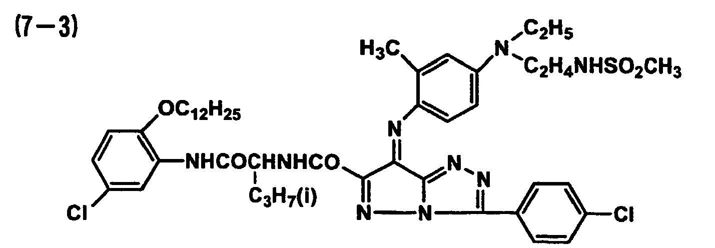

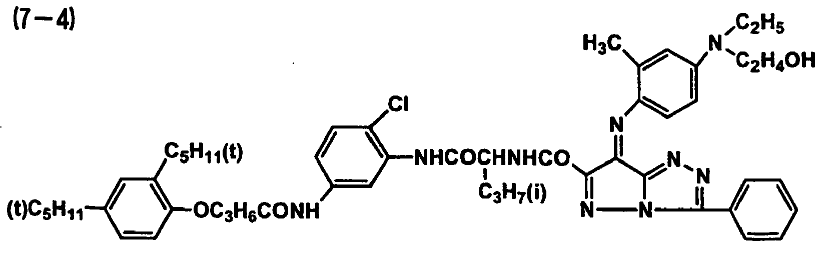

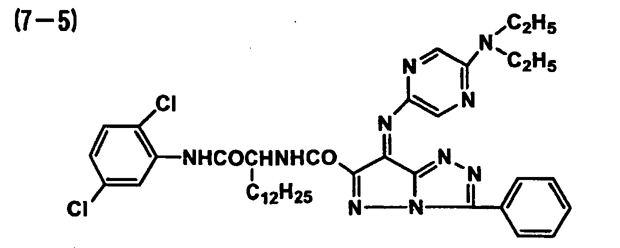

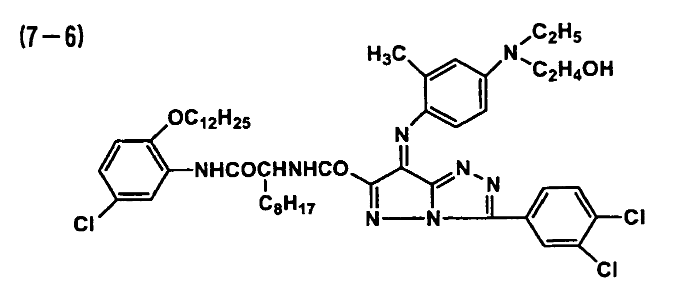

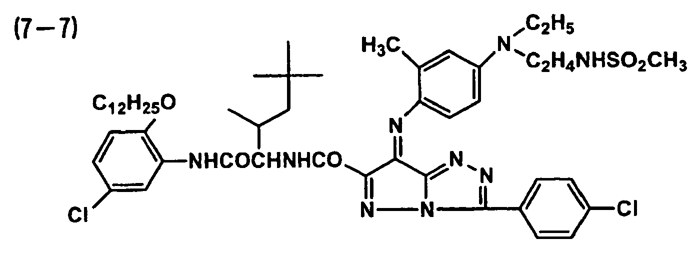

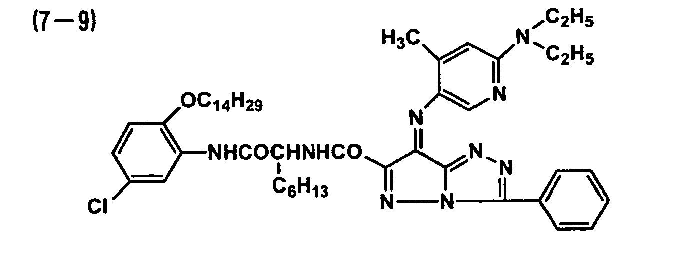

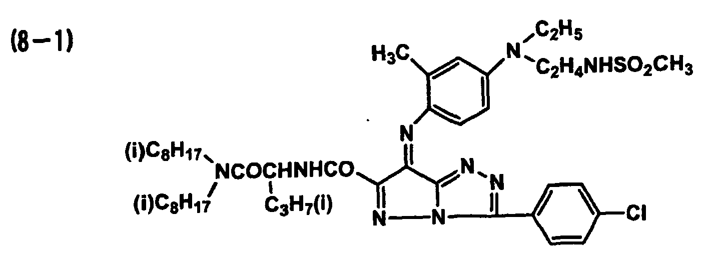

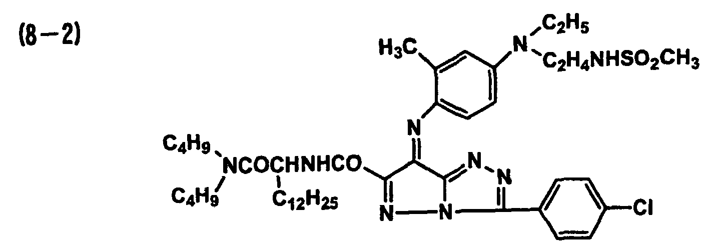

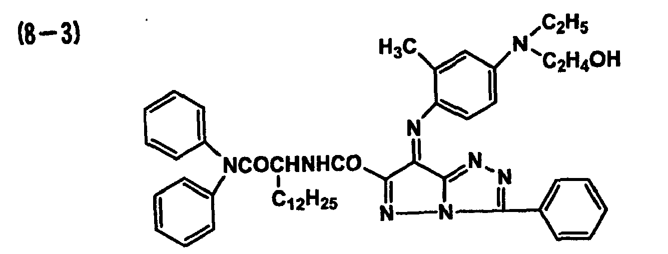

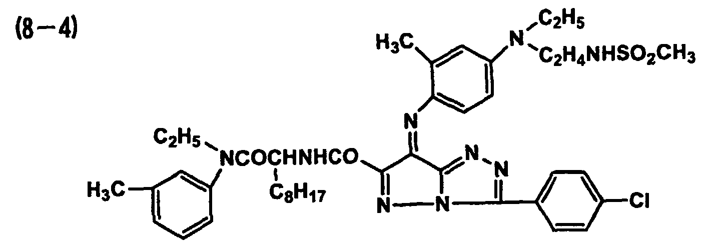

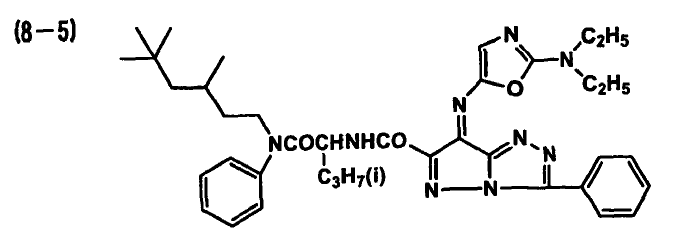

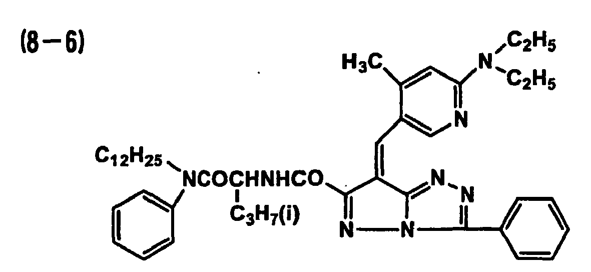

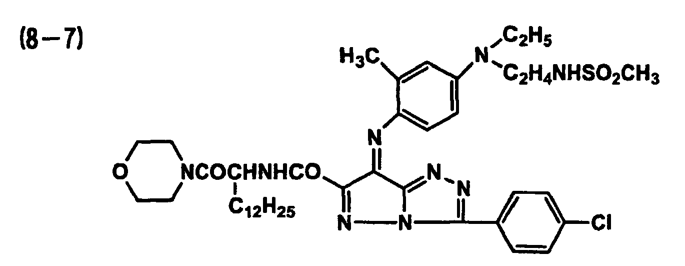

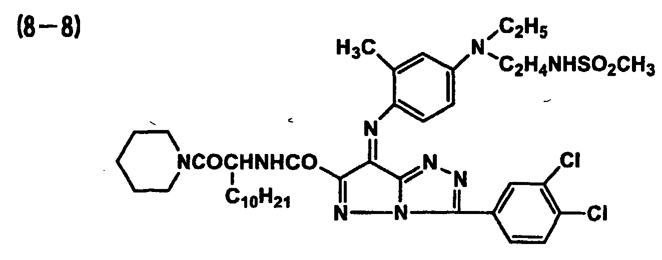

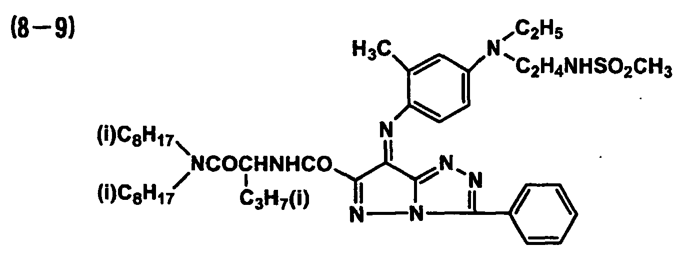

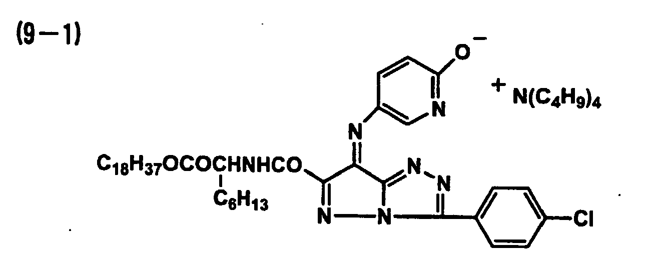

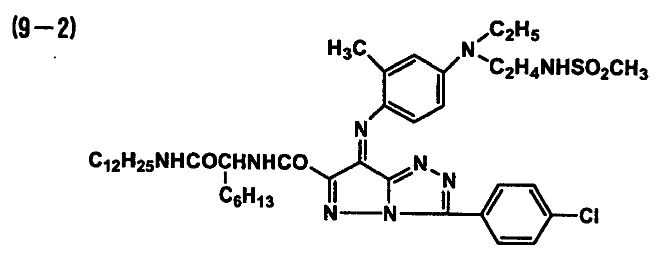

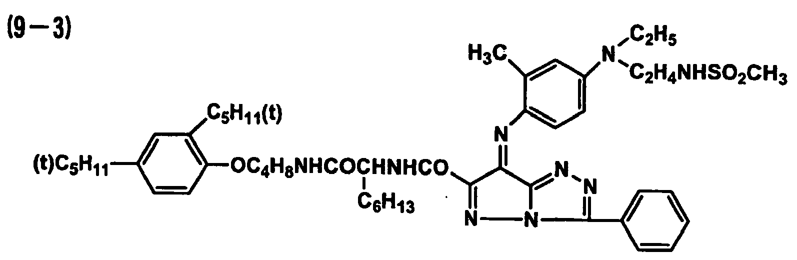

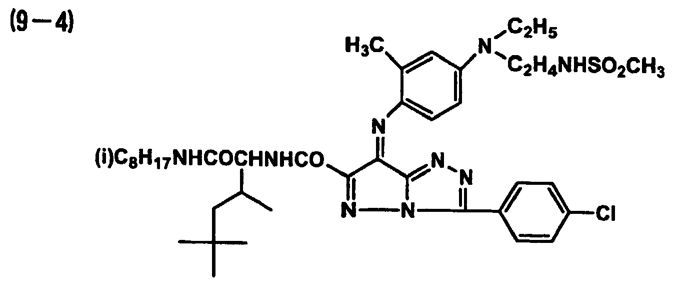

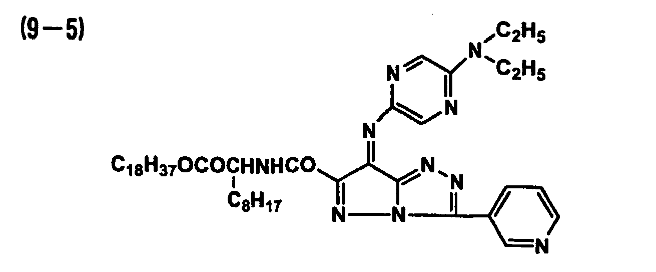

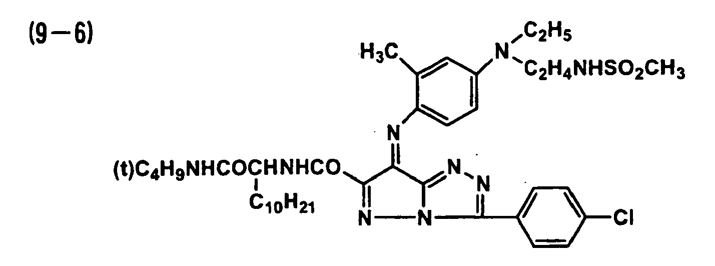

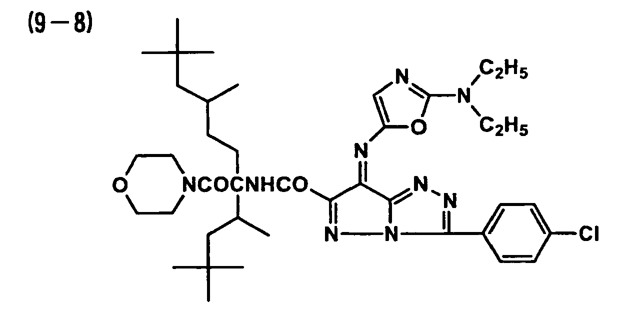

Examples of syntheses of the couplers that may be used in the invention are described below.

### i) Synthesis of exemplified compound 2-5

Compound 2-5 was synthesized in accordance with the following scheme 1 :

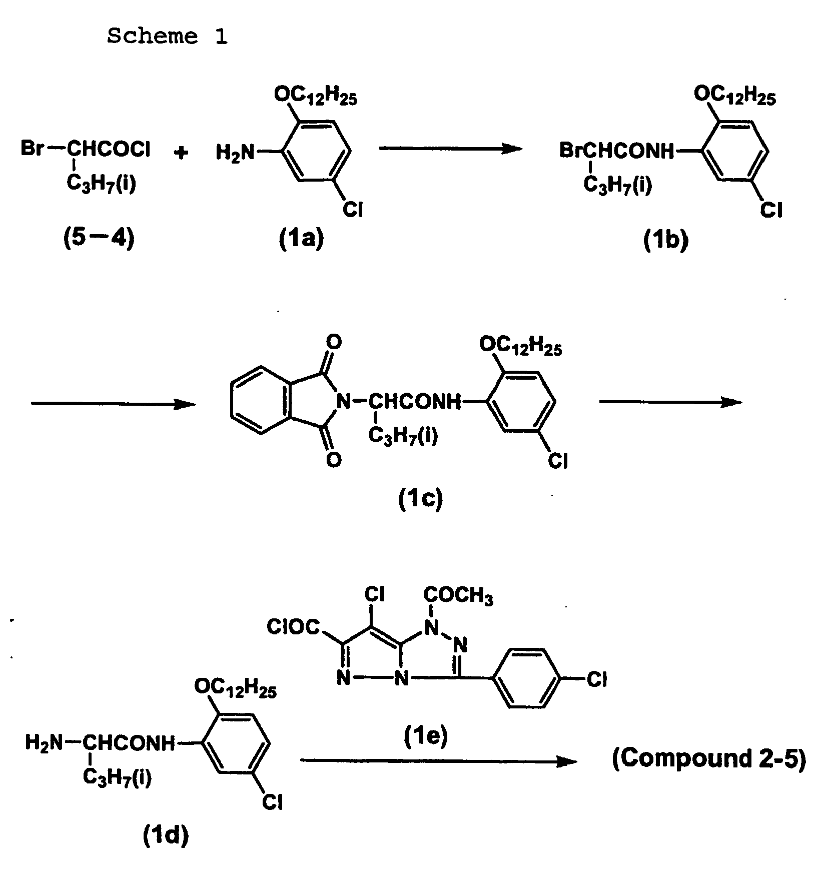

### Synthesis of intermediate (1b)

Compound (1a) of 31.1 g (0.10 ml) was dissolved in 150 ml of ethyl acetate and further thereto was added 23.7 g (0.3 mol) of pyridine. To this solution 20.9 g (0.105 mol) of exemplified compound (5-4) was added dropwise. After completion of addition, the reaction mixture was stirred for 2 hr. at the same temperature and after completion of reaction, aqueous hydrochloric acid was added and an organic layer was extracted. The extract was washed with water, dried and solvents were distilled under reduced pressure to obtain 47.5 g of intermediate (1b). The intermediate (1b) was used in the subsequent step, without being purified.

### Synthesis of intermediate (1c)

The intermediate (1b) of 47.5 g was dissolved in 90 ml of N,N-dimethylformamide and 19.4 g (0.105 mol) of potassium phthalimide was added thereto and allowed to react at ca. 90° C for 4 hr. After completion of reaction, the reaction product was extracted by adding ethyl acetate and water, dried and solvents were distilled under reduced pressure. The thus obtained residue was recrystallized in methanol to obtained intermediate (1c) of 46.0 g (yield of 85%).

### Synthesis of intermediate (1d)

The intermediate (1c) of 54.1 g (0.10 mol) was dispersed in 200 ml of ethanol and 7.51 g (0.15 mol) of hydrated hydrazine was added thereto and heated for 3 hr. under reflux. After completion of reaction, the reaction was allowed to stand for cooling, 10.3 ml (0.12 mol) of concentrated hydrochloric acid was added and stirred for 30 min., and crystals were filtered. Thereafter, solvents of the filtrate was distilled under reduced pressure and the residue was added with ethyl acetate and water, neutralized, and an organic layer was extracted therefrom. The extract was washed, dried and solvents were distilled under reduced pressure to obtain intermediate (1d) of 40.3 g (yield of 98%). The obtained intermediate (1d) was used in the subsequent step, without being purified.

### Synthesis of exemplified compound 2-5

Intermediate (1e) of 35.8 g (0.10 mol) was dispersed in 200 ml of ethyl acetate and 41.1 g (0.10 mol) of the intermediate (1d) was added thereto. Then, 20.2 g (0.20 mol) of triethylamine was added and reacted for 3 hr at room temperature. Subsequently, 24.4 ml (0.40 mol) of 28% ammonia water was added and further reacted for 3 hr. After completion of reaction, the reaction mixture was neutralized and an organic layer was extracted, washed with water, dried and solvent was distilled. The obtained residue was recrystallized in ethanol to obtained intended compound 2-5 of 62.8 g (yield of 91%). The melting point was 145 to 147° C and the structure was identified by proton NMR spectrum and mass spectrum.

### ii) Synthesis of exemplified compound 3-5

Compound 3-5 was synthesized in accordance with the following scheme 2:

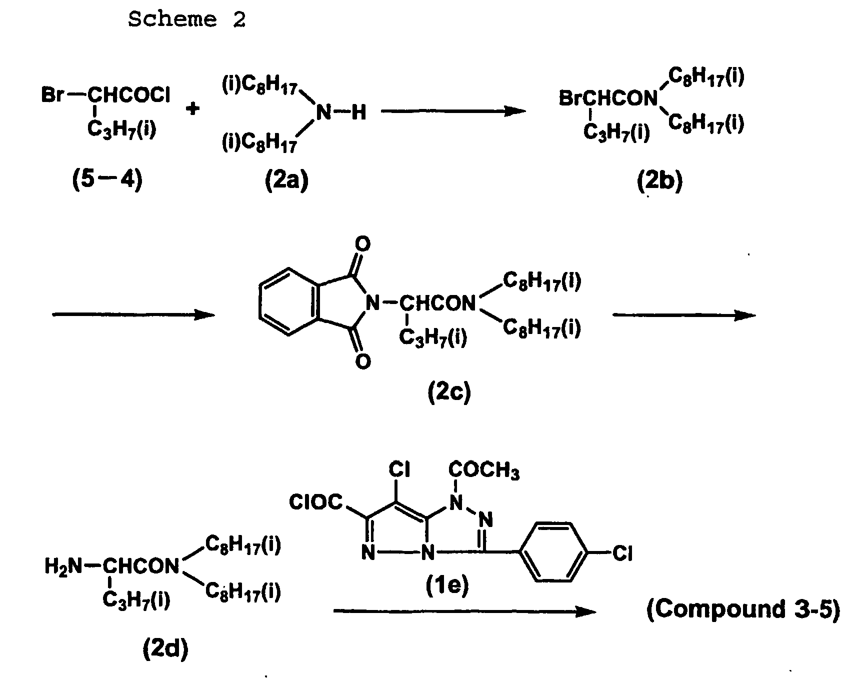

### Synthesis of intermediate (2b)

Compound (2a) of 24.1 g (0.10 ml) was dissolved in 150 ml of ethyl acetate and further thereto was added 23.7 g (0.3 mol) of pyridine. To this solution 20.9 g (0.105 mol) of compound (5-4) was added dropwise. After completion of addition, the reaction mixture was stirred for 2 hr. at the same temperature and after completion of reaction, aqueous hydrochloric acid was added and an organic layer was extracted. The extract was washed with water, dried and solvents was distilled under reduced pressure to obtain 40.4 g of intermediate (2b). The intermediate (2b) was used in the subsequent step, without being purified.

### Synthesis of intermediate (2c)

The intermediate (2b) of 40.4 g (0.10 mol) was dissolved in 80 ml of N,N-dimethylformamide and 19.4 g (0.105 mol) of potassium phthalimide was added thereto and allowed to react at ca. 90° C for 4 hr. After completion of reaction, the reaction product was extracted by adding ethyl acetate and water, dried and solvents were distilled under reduced pressure. The thus obtained residue was recrystallized in methanol to obtained intermediate (2c) of 38.6 g (yield of 82%).

### Synthesis of intermediate (2d)

The intermediate (2c) of 47.0 g (0.10 mol) was dispersed in 200 ml of ethanol and 7.51 g (0.15 mol) of hydrated hydrazine was added thereto and heated for 3 hr. under reflux. After completion of reaction, the reaction was allowed to stand for cooling, 10.3 ml (0.12 mol) of concentrated hydrochloric acid was added and stirred for 30 min., and crystals were filtered. Thereafter, solvents of the filtrate was distilled under reduced pressure and the residue was added with ethyl acetate and water, neutralized, and an organic layer was extracted therefrom. The extract was washed, dried and solvents were distilled under reduced pressure to obtain intermediate (2d) of 34.0 g (yield of 100%). The obtained intermediate (2d) was used in the subsequent step, without being purified.

### Synthesis of exemplified compound 3-5

Intermediate (1e) of 35.8 g (0.10 mol) was dispersed in 200 ml of ethyl acetate and 34.1 g (0.10 mol) of the intermediate (2d) was added thereto. Then, 20.2 g (0.20 mol) of triethylamine was added and reacted for 3 hr at room temperature. Subsequently, 24.4 ml (0.40 mol) of 28% ammonia water was added and further reacted for 3 hr. After completion of reaction, the reaction mixture was neutralized and an organic layer was extracted, washed with water, dried and solvent was distilled. The obtained residue was recrystallized in ethanol to obtained intended compound 3-5 of 55.2 g (yield of 89%). The melting point was 83 to 88° C and the structure was identified by proton NMR spectrum and mass spectrum.

### iii) Synthesis of exemplified compound 4-24

Compound 4-24 was synthesized in accordance with the following scheme 3:

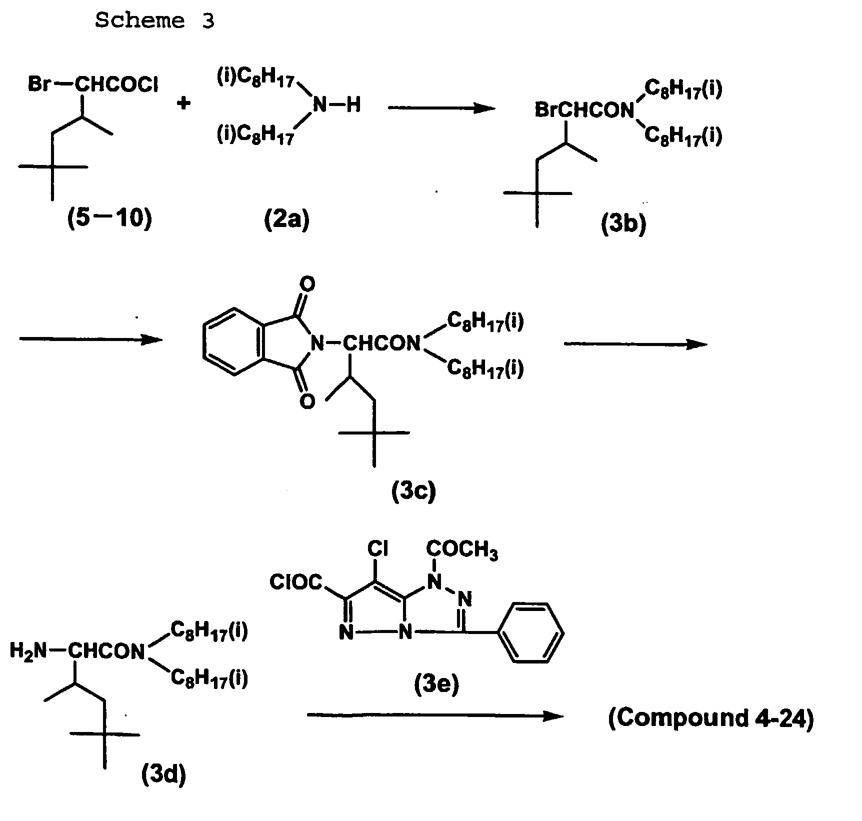

### Synthesis of intermediate (3b)

Compound (2a) of 24.1 g (0.10 ml) was dissolved in 150 ml of ethyl acetate and further thereto was added 23.7 g (0.3 mol) of pyridine. To this solution 25.6 g (0.105 mol) of compound (5-10) was added dropwise. After completion of addition, the reaction mixture was stirred for 2 hr. at the same temperature and after completion of reaction, aqueous hydrochloric acid was added and an organic layer was extracted. The extract was washed with water, dried and solvents was distilled under reduced pressure to obtain 46.1 g of intermediate (3b). The intermediate (3b) was used in the subsequent step, without being purified.

### Synthesis of intermediate (3c)

The intermediate (3b) of 46.1 g (0.10 mol) was dissolved in 80 ml of N,N-dimethylformamide and 19.4 g (0.105 mol) of potassium phthalimide was added thereto and allowed to react at ca. 90° C for 4 hr. After completion of reaction, the reaction product was extracted by adding ethyl acetate and water, dried and solvents were distilled under reduced pressure. The thus obtained residue was recrystallized in methanol to obtained intermediate (3c) of 49.5 g (yield of 94%).

### Synthesis of intermediate (3d)

The intermediate (3c) of 52.7 g (0.10 mol) was dispersed in 200 ml of ethanol and 7.51 g (0.15 mol) of hydrated hydrazine was added thereto and heated for 3 hr. under reflux. After completion of reaction, the reaction was allowed to stand for cooling, 10.3 ml (0.12 mol) of concentrated hydrochloric acid was added and stirred for 30 min., and crystals were filtered. Thereafter, solvents of the filtrate was distilled under reduced pressure and the residue was added with ethyl acetate and water, neutralized, and an organic layer was extracted therefrom. The extract was washed, dried and solvents were distilled under reduced pressure to obtain intermediate (3d) of 39.7 g (yield of 100%). The obtained intermediate (2d) was used in the subsequent step, without being purified.

### Synthesis of exemplified compound 4-24

Intermediate (3e) of 35.8 g (0.10 mol) was dispersed in 200 ml of ethyl acetate and 39.7 g (0.10 mol) of the intermediate (3d) was added thereto. Then, 20.2 g (0.20 mol) of triethylamine was added and reacted for 3 hr at room temperature. Subsequently, 24.4 ml (0.40 mol) of 28% ammonia water was added and further reacted for 3 hr. After completion of reaction, the reaction mixture was neutralized and an organic layer was extracted, washed with water, dried and solvent was distilled. The obtained residue was recrystallized in ethanol to obtain intended compound 4-24 of 57.7 g (yield of 90%). The structure was identified by proton NMR spectrum and mass spectrum.

### iv) Synthesis of exemplified compound 8-9

Compound 8-9 was synthesized in accordance with the following scheme 4:

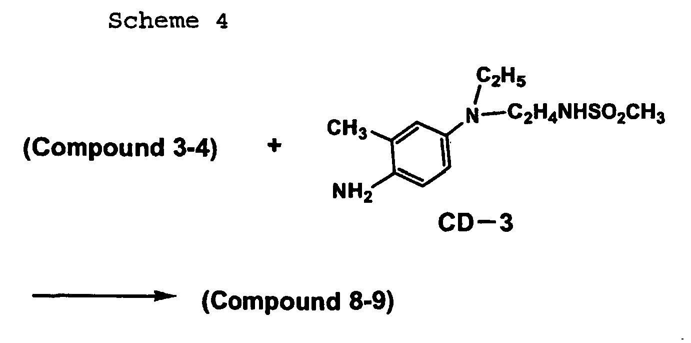

Exemplified compound (3-4) of 11.7 g (0.02 mol) and 120 ml of ethyl acetate were stirred at room temperature and thereto was added a separately prepared solution composed of 8.56 g (0.03 mol) of CD-3, 4.15 g (0.03 mol) of potassium carbonate, a small amount of sodium sulfite and 100 ml of water. A solution of 6.95 g (0.03 mol) of silver oxide dispersed in 50 ml of water was added dropwise for 20 min. After stirring for 3 hr. at room temperature and using diatomite, silver was filtered with suction and washed with ethyl acetate. The washing solution and filtrate were combined and washed with 1 mol/l hydrochloric acid, then, washed with water and saturated sodium chloride solution and dried using magnesium sulfate, thereafter, the solvent was concentrated. The product was purified by a silica gel column (solvent = toluene 3 : ethyl acetate 1) and recrystallized in a mixture solvent of toluene and hexane to obtain intended compound 8-9 of 3.2 g. The structure was identified by NMR and mass spectrum. Absorption spectra of compound 8-9 in methanol and in ethyl acetate are shown in Fig. 1.

The couplers described herein can be used in an amount of from 1x10⁻³ to 1 mol, and preferably from 1x10⁻² to 8x10⁻¹ mol per mol silver halide. The couplers may be used in combination with other kinds of couplers. Methods and techniques used in conventional couplers are also applicable to the couplers described herein. The couplers are employed in color photographic materials, such as color negative film, positive film and color print paper.

Photographic materials, such as color print paper, using the couplers, may be those for single-color or multicolor use. In multicolor photographic materials, the couplers are usually incorporated into a red-sensitive silver halide emulsion layer. The multicolor photographic material comprises dye image forming component units having sensitivity in each of three primary color regions of spectrum. Each of the component units comprises a single-layered or multi-layered emulsion layer having sensitivity within a given region of spectrum. The component layers of the photographic material, including the image forming component units can be arranged in any order, as is known in the art.

Typical multicolor photographic material comprises a support provided thereon a cyan dye image forming component unit having a red-sensitive silver halide emulsion layer containing a cyan coupler, a magenta dye image forming component unit having a green-sensitive silver halide emulsion layer containing a magenta coupler and a yellow dye image forming component unit having a blue-sensitive silver halide emulsion layer containing a yellow coupler. The photographic material may further be provided with additional layer(s), such as a filter layer, interlayer, protective layer and sublayer.

There can be employed commonly known methods to incorporate the couplers described herein. For example, after the couplers are dissolved alone or in combination in a high boiling organic solvent having a boiling point of 175° C or higher, such as tricresyl phosphate or dibutyl phthalate or a low boiling solvent, such as butyl acetate or butyl propionate, or in a mixture thereof, the resulting solution is mixed with an aqueous gelatin solution containing a surfactant and emulsified by a high-speed rotating mixer or colloid mill and the emulsion is incorporated in a silver halide emulsion to obtain a silver halide emulsion.

Silver halide used in the photographic materials of the invention is not specifically limited, and may include silver chloride, silver bromochloride and silver iodobromochloride. In this case, there may be a mixture of silver chloride and silver bromide. In cases where the silver halide emulsion is used in color print paper, rapid-developability is required, so that the halide composition of the silver halide preferably contains chlorine, and silver chloride, silver bromochloride or silver iodobromochloride, each of which contains 1 mol% or more chloride, are specifically preferred. These silver halide emulsions are subjected to chemical sensitization in accordance with conventional methods and are also spectrally sensitized to the desired wavelength region.

Compounds known in the art as an antifoggant or stabilizer may be incorporated into the silver halide emulsion to prevent fogging caused in the process of preparing photographic material, after storage thereof or during photographic processing, and/or to stably maintain photographic performance.

In the color photographic material of the invention, various additives can be used, including, for example, anti-color staining agent, dye image stabilizer, UV absorber, matting agent and surfactant, as described in Research Disclosure Vol. 176, page 22-31 (December, 1978).

The photographic material of the invention can be subjected to commonly known color development to form color images, followed by bleaching and fixing. Bleaching may be concurrently conducted with fixing. After fixing, washing is usually conducted. Alternatively, a stabilization treatment may be conducted in place of washing.

The photographic material of the invention may contain a color developing agent or its precursor in a hydrophilic colloid layer, which can be processed in an alkaline activator bath.

### EXAMPLES

The present invention will be described based on examples but embodiments of the invention are by no means limited to these.

### Example 1

To 1 g of each of couplers and comparative couplers, as shown in Table 1, tricresyl phosphate of an equal weight to the coupler and ethyl acetate of three times the weight of the coupler were added and the temperature at which the coupler was completely dissolved was determined. Results thereof are shown in Table 1. Structures of comparative couplers a to e are respectively shown below.

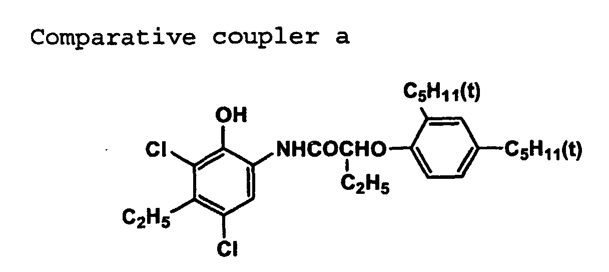

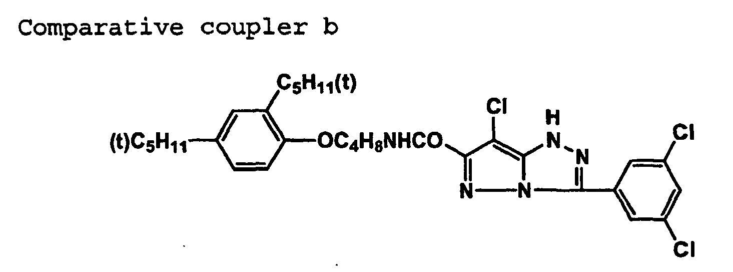

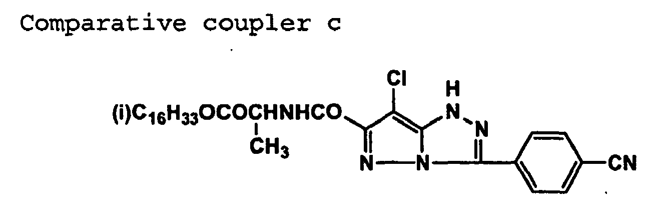

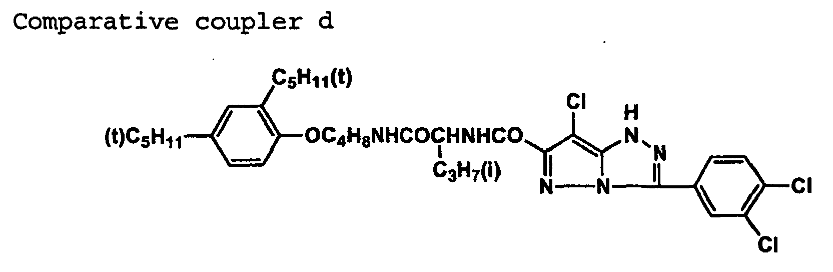

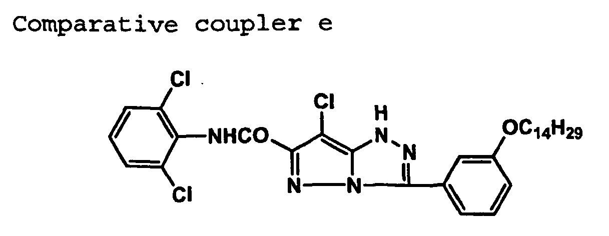

**Table 1**

| Experiment No. | Coupler | Dissolution Temperature (°C) |
|---|---|---|
| 1 Control | 2-4 | 30 |
| 2 Control | 3-4 | Room temperature |
| 3 Control | 3-5 | 30 |
| 4 Control | 4-24 | Room temperature |
| 5 (Comp.) | b | 60 |
| 6 (Comp.) | d | 60 |

As apparent from Table 1, it was proved that couplers of formulae (2), (3) or (4) were superior to comparative couplers, in terms of solubility in organic solvents (including high boiling organic solvent and low boiling organic solvent).

### Example 2

On a paper support laminated on both sides with polyethylene, the following layers were coated in this order from the support to prepare red-sensitive color photographic material sample No. 1. The coating amount of a compound was represented in terms of per m² and silver halide was represented by equivalent converted to silver.

### 1st layer: Emulsion layer

Red-sensitive emulsion layer comprising 1.3 g of gelatin, 0.21 mol of red-sensitive silver bromochloride emulsion (containing 99.5 mol% chloride) and comparative coupler a (9.1x10⁻⁴ mol) dissolved in 0.45 g of dioctyl phthalate;

### 2nd layer: Protective layer

Protective layer containing 0.50 g of gelatin, in which 2,4-dichloro-6-hydroxy-s-triazine sodium salt was incorporated as a hardener in an amount of 0.017 g per g of gelatin.

Further, samples No. 2 through No. 18 were prepared similarly to sample No. 1, except that comparative coupler a was replaced by a coupler shown in Table 2 (in an amount of 50 mol% of comparative coupler a), and the red-sensitive silver bromochloride emulsion was used in an amount of 70 mol% of sample No. 1. The thus obtained samples Nos. 1 through 18 were each stepwise exposed to light through an optical wedge in accordance with the conventional manner and processed according to the following steps:

### Process

| Step | Temperature | Time |
|---|---|---|
| Color developing | 35.0±0.3° C | 45 sec. |
| Bleach-fixing | 35.0±0.5° C | 45 sec. |
| Stabilizing | 30 to 34° C | 90 sec. |
| Drying | 60 to 80° C | 60 sec. |

Compositions of processing solutions used in the respective steps are as follows. Color developer solution

| | |
|---|---|
| Pure water | 800 ml |
| Triethanolamine | 10 g |
| N,N-diethylhydroxylamine | 5 g |
| Potassium bromide | 0.02 g |
| Potassium chloride | 2 g |
| Potassium sulfite | 0.3 g |
| 1-Hydroxyethylidene-1,1-diphosphonic acid | 1.0 g |
| Ethylenediaminetetraacetic acid | 1.0 g |
| Disodium catechol-3,5-disulfonate | 1.0 g |
| Diethylene glycol | 10 g |
| N-Ethyl-N-β-methanesulfoneamidoethyl-3-methyl-aminoaniline sulfate | 4.5 g |
| Brightener (4,4'-diaminostilbenesulfonic acid derivative) | 1.0 g |
| Potassium carbonate | 27 g |

Water was added to make 1 liter and the pH adjusted to 10.10.

### Bleach-fixer solution

| | |
|---|---|
| Ethylenediaminetetraacetate, ferric ammonium, dihydrate | 60 g |
| Ethylenediaminetetraacetic acid | 3 g |
| Ammonium thiosulfate (70% aqueous solution) | 100 ml |
| Ammonium sulfite (40% aqueous solution) | 27.5 ml |

Water was added to make 1 liter and the pH was adjusted to 5.7 with potassium carbonate or glacial acetic acid.

### Stabilizer solution

| | |
|---|---|
| 5-Chloro-2-methyl-4-isothiazoline-3-one | 0.2 g |
| 1,2-Benzisothiazoline-3-one | 0.3 g |
| Ethylene glycol | 1.0 g |
| 1-Hydroxyethylidene-1,1-diphosphonic acid | 2.0 g |
| o-Phenylphenol sodium salt | 1.0 g |
| Ethylenediaminetetraacetic acid | 1.0 g |
| Ammonium hydrate (20% aqueous solution) | 3.0 g |
| Brightener (4,4'-diaminostilbenesulfonic | |
| acid derivative) | 1.5 g |

Water was added to make 1 liter and the pH was adjusted to 7.0 with sulfuric acid or potassium hydroxide.

The thus processed samples Nos. 1 through 18 were subjected to densitometry using a densitometer (Type KD-7, available from Konica Corp.) to determine a maximum density (also denoted as Dmax). Furthermore, the processed samples were exposed in a Xenon Fade-O-meter (90,000 lux) for 24 hrs. and then measured with respect to yellow stain density. Results are shown in Table 2.

**Table 2**

| Sample No. | Coupler | Dmax | Yellow Stain |
|---|---|---|---|
| 1 | a | 2.30 | 0.01 |
| 2 | b | 2.08 | 0.02 |
| 3 | c | 2.04 | 0.02 |
| 4 | d | 2.10 | 0.01 |
| 5 | e | 2.19 | 0.02 |
| 6 | 2―4 | 2.38 | 0.01 |
| 7 | 2―5 | 2.37 | 0.01 |
| 8 | 2―7 | 2.36 | 0.01 |
| 9 | 2―11 | 2.33 | 0.01 |
| 10 | 3―4 | 2.41 | 0.00 |
| 11 | 3―5 | 2.39 | 0.01 |
| 12 | 3―17 | 2.34 | 0.01 |
| 13 | 3―24 | 2.33 | 0.01 |
| 14 | 3―29 | 2.40 | 0.01 |
| 15 | 4―4 | 2.42 | 0.01 |
| 16 | 4―12 | 2.38 | 0.01 |
| 17 | 4―18 | 2.36 | 0.01 |
| 18 | 4―24 | 2.40 | 0.00 |

As apparent from Table 2, it was proved that samples using couplers of formulae (2), (3) or (4) exhibited Dmax higher than samples using comparative couplers, even when used in amounts of 50 mol% coupler and 70 mol% silver halide, preventing yellow stains caused after Xe exposure.

### Example 3

On a paper support laminated, on one side of paper, with polyethylene and laminated, on the other side, with polyethylene containing titanium oxide, the following layers were coated on the titanium oxide containing polyethylene layer side to prepare a multilayer color photographic material sample No. 19. Coating solutions were prepared as follows.

### 1st layer coating solution:

To 26.7 g of yellow coupler (Y-1), 10.0 g of dye image stabilizer (ST-1), 6.67 g of dye image stabilizer (ST-2), 0.67 g of additive (HQ-1), 0.6 g of anti-irradiation dye (AI-3), and 6.67 g of high boiling solvent (DNP) was added 60 ml of ethyl acetate. Using a ultrasonic homogenizer, the resulting solution was dispersed in 220 ml of an aqueous 10% gelatin solution containing 7 ml of an aqueous 20% surfactant (SU-1) solution to obtain a yellow coupler dispersion. The obtained dispersion was mixed with the blue-sensitive silver halide emulsion (containing 8.68 g of silver) to prepare a 1st layer coating solution. Coating solutions for the 2nd layer to 7th layer were each prepared similarly to the 1st layer coating solution, and each coating solution was coated so as to have a coating amount as shown below.

Hardeners (H-1) and (H-2) were incorporated into the 2nd and 4th layers. There were also incorporated surfactants, (SU-2) and (SU-3) to adjust surface tension. Antiseptic DI-1 was further incorporated. Unless otherwise noted, the coating amounts of respective compounds were represented in terms of grams per m².

| Layer | Constitution | Amount (g/m²) |
|---|---|---|
| 7th Layer | Gelatin | 1.00 |
| (Protective layer) | DIDP | 0.005 |
| | Additive (HQ-2) | 0.002 |
| | Additive (HQ-3) | 0.002 |
| | Additive (HQ-4) | 0.004 |
| | Additive (HQ-5) | 0.02 |
| | Compound (F-1) | 0.002 |
| 6th Layer | Gelatin | 0.40 |
| (UV absorbing layer) | UV absorbent (UV-1) | 0.10 |
| | UV absorbent (UV-2) | 0.04 |
| | UV Absorbent (UV-3) | 0.16 |
| | Additive (HQ-5) | 0.04 |
| | DNP | 0.20 |
| | PVP | 0.03 |
| | Antiirradiation dye (AI-2) | 0.02 |
| | Antiirradiation dye (AI-4) | 0.01 |
| 5th Layer | Gelatin | 1.30 |
| (Red-sensitive layer) | Red-sensitive emulsion (Em-R) | 0.21 |
| | Cyan coupler (C-1) | 0.17 |
| | Cyan coupler (C-2) | 0.25 |
| | Dye image stabilizer (ST-1) | 0.20 |
| | Antistaining agent (HQ-1) | 0.01 |
| | HBS-1 | 0.20 |
| | DOP | 0.20 |
| 4th Layer | Gelatin | 0.94 |
| (UV absorbing layer) | UV absorbent (UV-1) | 0.28 |
| | UV absorbent (UV-2) | 0.09 |
| | UV absorbent (UV-3) | 0.38 |
| | Additive (HQ-5) | 0.10 |
| | DNP | 0.40 |
| 3rd Layer | Gelatin | 1.40 |
| (Green-sensitive layer) | Green-sensitive Emulsion (Em-G) | 0.17 |
| | Magenta coupler (M-1) | 0.23 |
| | Dye image stabilizer (ST-3) | 0.20 |
| | Dye image stabilizer (ST-4) | 0.17 |
| | DIDP | 0.13 |
| | DBP | 0.13 |
| | Antiirradiation dye (AI-1) | 0.01 |
| 2nd layer | Gelatin | 1.20 |
| (Interlayer) | Antistaining agent (HQ-2) | 0.03 |
| | Antistaining agent (HQ-3) | 0.03 |
| | Antistaining agent (HQ-4) | 0.05 |
| | Antistaining agent (HQ-5) | 0.23 |
| | DIDP | 0.06 |
| | Compound (F-1) | 0.002 |
| 1st layer | Gelatin | 1.20 |
| (Blue-sensitive layer) | Blue-sensitive Emulsion (Em-B) | 0.26 |
| | Yellow coupler (Y-1) | 0.80 |
| | Dye image stabilizer (ST-1) | 0.30 |
| | Dye image stabilizer (ST-2) | 0.20 |
| | Additive (HQ-1) | 0.02 |
| | Antiirradiation dye (AI-3) | 0.01 |
| | DNP | 0.20 |
| Support | Polyethylene-laminated paper | |

The coating amount of a silver halide emulsion was represented in terms of equivalent converted to silver. 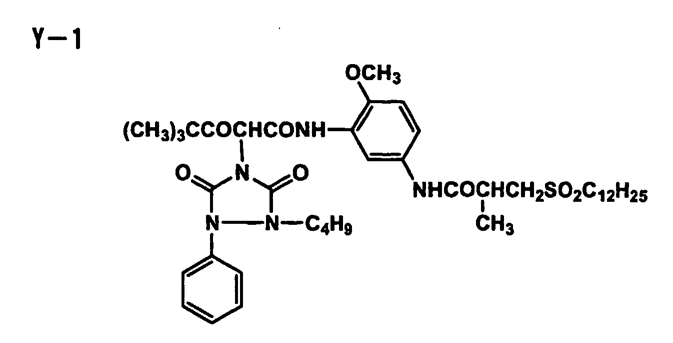 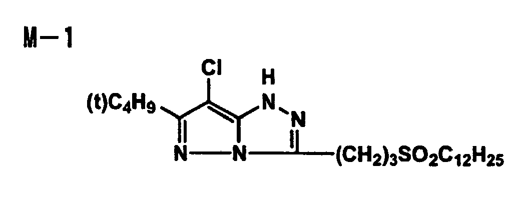 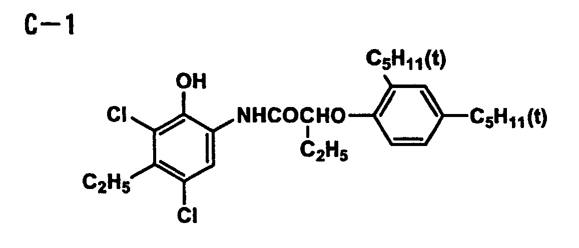 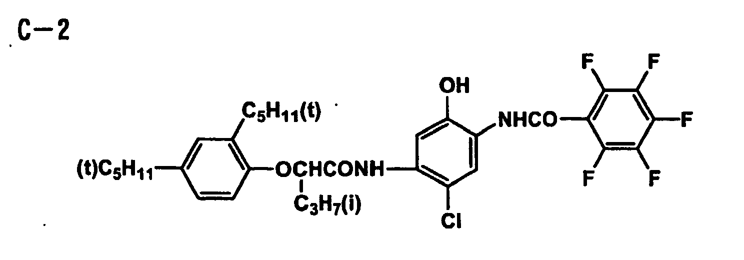 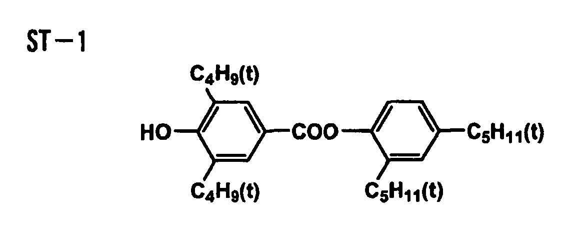 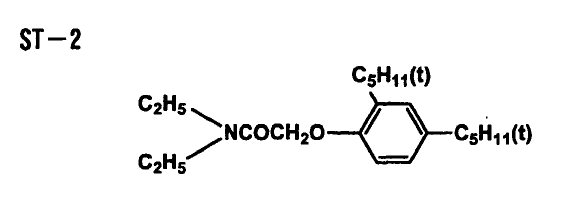 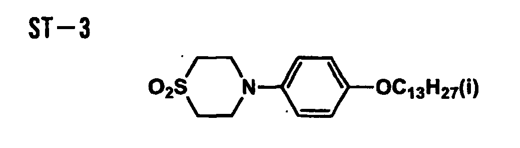 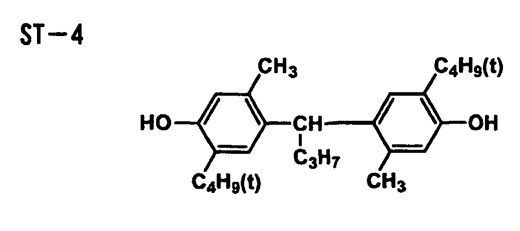 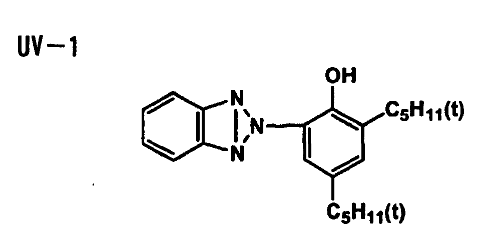 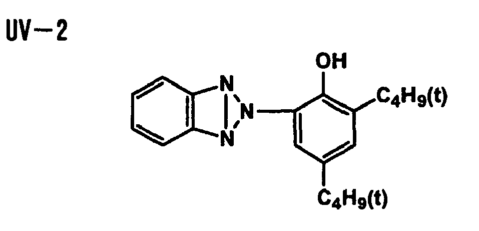 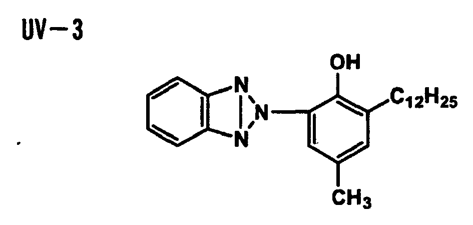
DBP: Dibutyl phthalate
DOP: Dioctyl phthalate
DNP: Dinonyl phthalate
DIDP: Diisodecyl phthalate
PVP: Polyvinyl pyrrolidine

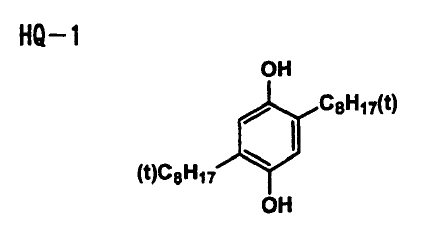

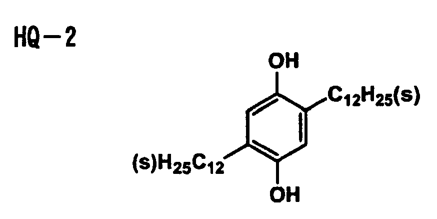

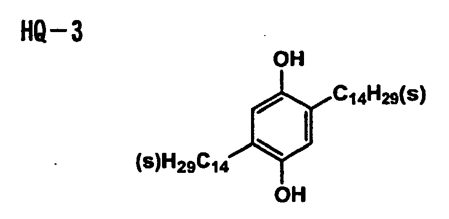

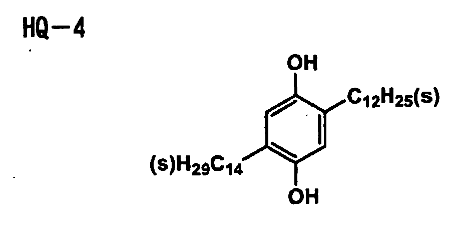

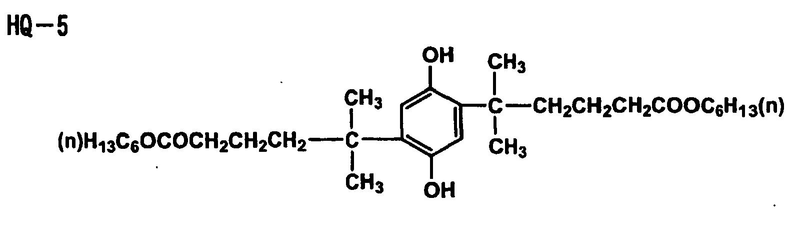

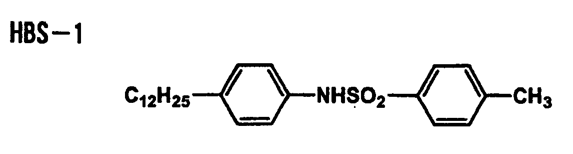

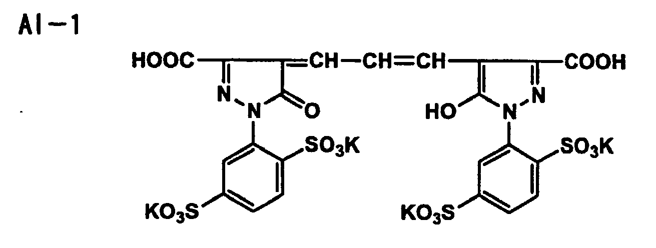

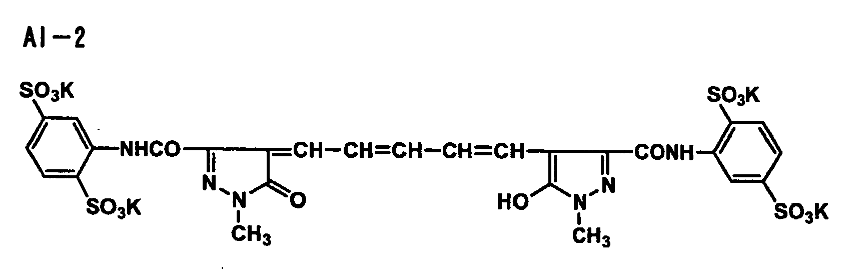

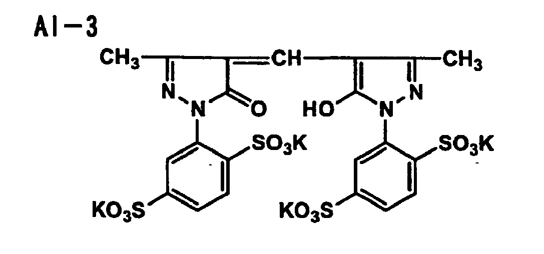

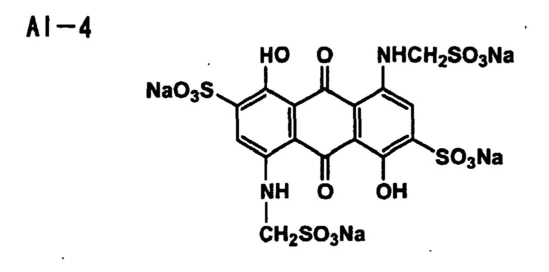

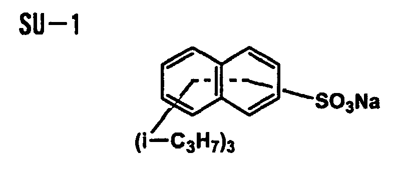

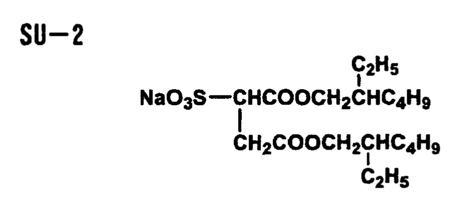

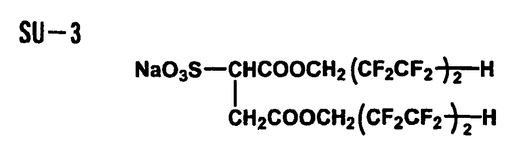

H―1 C(CH₂SO₂CH=CH₂)₄

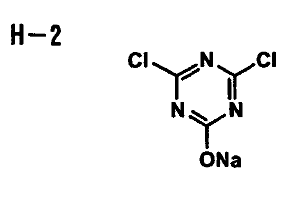

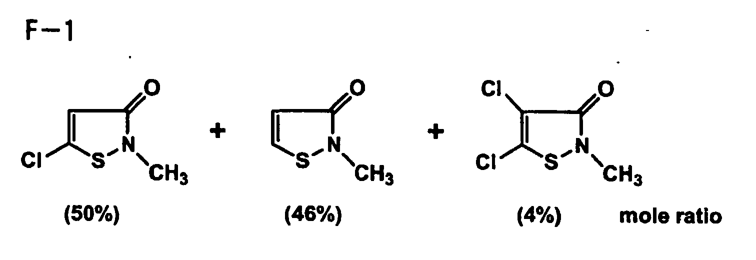

### Preparation of blue-sensitive silver halide emulsion

To 1000 ml of aqueous 2% gelatin solution kept at 40° C were simultaneously added the following solutions (Solutions A and B) in 30 min., while being maintained at a pAg of 6.5 and pH of 3.0, and further thereto were added Solutions C1 and D1 in 180 min., while being maintained at a pAg of 7.3 and pH of 5.5. The pH was controlled with sulfuric acid or sodium hydroxide, and the pAg was controlled using a control solution, which was an aqueous solution comprised of sodium chloride and potassium bromide (Cl:Br = 99.8:0.2) and the concentration of which was 0.1 mol/l in mixing solutions A and B and 1 mol/l in mixing solutions C and D.

| Solution A | |
|---|---|
| Sodium chloride | 3.42 g |
| Potassium bromide | 0.03 g |
| Water to make | 200 ml |

| Solution B | |
|---|---|
| Silver nitrate | 10 g |
| Water to make | 200 ml |

| Solution C | |
|---|---|
| Sodium chloride | 102.7 g |
| Potassium bromide | 1.0 g |
| Water to make | 600 ml |

| Solution D | |
|---|---|
| Silver nitrate | 300 g |
| Water to make | 600 ml |

After completing the addition, the resulting emulsion was desalted using a 5% aqueous solution of Demol N (produced by Kao-Atlas) and aqueous 20% magnesium sulfate solution, and re-dispersed in a gelatin aqueous solution to obtain a monodisperse cubic grain emulsion (EMP-1) having an average grain size of 0.85 µm, a coefficient of variation of grain size (S/R) of 0.07 and a chloride content of 99.5 mol%.

The emulsion, EMP-1 was chemically sensitized at 50° C for 90 min., using the following compounds to obtain blue-sensitive silver halide emulsion (Em-B).

| | |
|---|---|
| Sodium thiosulfate | 0.8 mg/mol AgX |
| Chloroauric acid | 0.5 mg/mol AgX |
| Stabilizer STAB-1 | 6x10⁻⁴ mol/mol AgX |
| Sensitizing dye BS-1 | 4x10⁻⁴ mol/mol AgX |
| Sensitizing dye BS-2 | 1x10⁻⁴ mol/mol AgX |

### Preparation of green-sensitive silver halide emulsion

Monodisperse cubic grain emulsions, EMP-2 having an average grain size of 0.43 µm, a variation coefficient (S/R) of 0.08 and a chloride content of 99.5 mol% was prepared in the same manner as in preparation of EMP-1, except that an adding time of Solutions A and B, and that of Solution C and D were respectively varied.

The emulsion, EMP-2 was optimally chemical-sensitized at 55° C for 120 min. using the following compounds to obtain a green-sensitive silver halide emulsion (Em-G).

| | |
|---|---|
| Sodium thiosulfate | 1.5 mg/mol AgX |
| Chloroauric acid | 1.0 mg /mol AgX |
| Stabilizer STAB-1 | 6x10⁻⁴ mol/mol AgX |
| Sensitizing dye GS-1 | 4x10⁻⁴ mol/mol AgX |

### Preparation of red-sensitive silver halide emulsion

Monodisperse cubic grain emulsion EMP-3 having an average grain size of 0.50 µm, a variation coefficient (S/R) of 0.08 and a chloride content of 99.5 mol% were prepared in the same manner as in preparation of EMP-1, except that an adding time of Solutions A and B, and that of Solution C and D were respectively varied.

Emulsion EMP-3 was optimally chemically sensitized at 60° C for 90 min. using the following compounds to obtain a red-sensitive silver halide emulsion (EM-P).

| | |
|---|---|
| Sodium thiosulfate | 1.8 mg/mol AgX |
| Chloroauric acid | 2.0 mg/mol AgX |
| Stabilizer STAB-1 | 6x10⁻⁴ mol/mol AgX |
| Sensitizing dye RS-1 | 1x10⁻⁴ mol/mol AgX |

In the foregoing, the variation coefficient (S/R) was calculated from standard deviation (S) and average grain size (R), in which S = [Σ(Ri-R)²/ Σni]^{1/2}, Ri is grain size and ni is the number of grains having grain size of Ri.

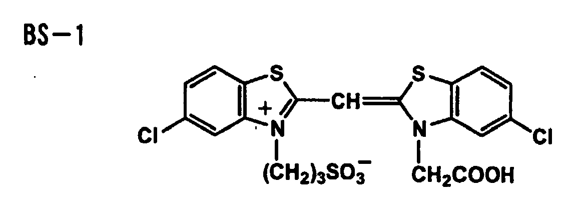

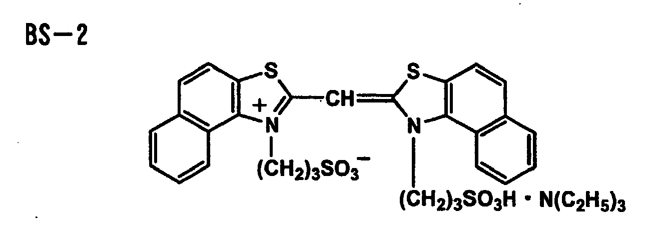

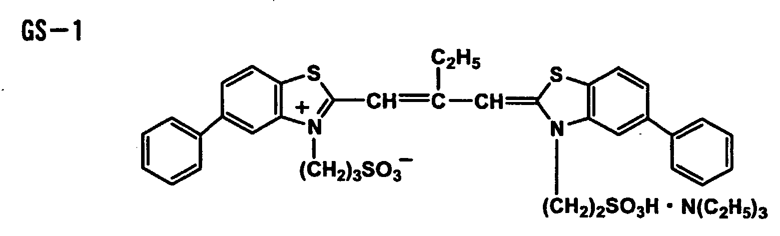

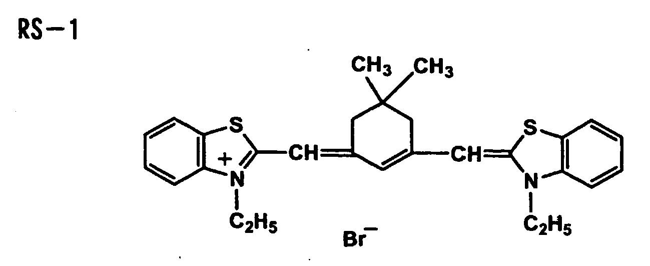

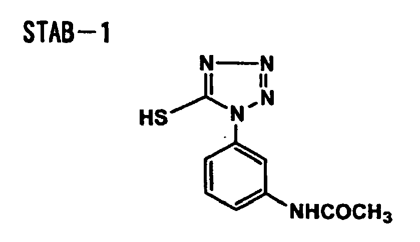

Photographic material sample Nos. 20 through 31 were prepared similarly to sample No. 19, except that cyan couplers C-1 and C-2 used in the 5^{th} layer were replaced by a coupler shown in Table 5, in an amount of 50 mol% of the sum of C-1 and C-2, and the red-sensitive silver bromochloride emulsion was changed to 70 mol%. The thus obtained samples were each exposed and processed similarly to Example 1, and further subjected to densitometry to determined the maximum density (Dmax) of the red-sensitive layer.

Furthermore, samples were evaluated with respect to color reproduction in the following manner. First, Macbeth color checker was photographed using color negative film (Konica Color Centuria 400, available from Konica Corp.) and a camera (Konica FT-1 MOTOR, product by Konica Corp.). Then, photographed film was processed in color negative processing (CNK-4, available from Konica Corp.) and obtained negative images were printed on each of the foregoing sample Nos. 19 through 31 with a size of 82x117 mm, using Konica Color Printer CL-P 2000 (available from Konica Corp.), in which the printing condition was set for each of the samples so that gray on the color checker was reproduced as gray in the print. The thus obtained prints were visually evaluated with respect to color reproduction and blackness. Evaluation was conducted by five persons and marked in accordance with the number of persons approving the print, as below:

| | |
|---|---|
| 5 marks: | 5 persons |
| 4 marks: | 4 persons |
| 3 marks: | 3 persons |
| 2 marks: | 2 persons |
| 1 mark: | 0 to 1 person. |

Results are shown in Table 3.

**Table 3**

| Sample No. | Cyan Coupler | Dmax | Color Reproduction | | | Blackness | Remark |
|---|---|---|---|---|---|---|---|
| | | | Cyan | Blue | Green | | |
| 19 | C-1/C-2 | 2.29 | 3 | 3 | 3 | 3 | Comp. |
| 20 | b | 2.08 | 4 | 5 | 3 | 2 | Comp. |
| 21 | d | 2.10 | 4 | 5 | 4 | 2 | Comp. |
| 22 | 2-5 | 2.37 | 5 | 5 | 5 | 5 | Inv. |
| 23 | 2-14 | 2.32 | 5 | 5 | 5 | 5 | Inv. |
| 24 | 2-17 | 2.35 | 5 | 5 | 5 | 5 | Inv. |
| 25 | 3-5 | 2.39 | 5 | 5 | 5 | 5 | Inv. |
| 26 | 3-8 | 2.36 | 5 | 5 | 5 | 5 | Inv. |
| 27 | 3-13 | 2.32 | 5 | 5 | 4 | 5 | Inv. |
| 28 | 3-28 | 2.32 | 5 | 5 | 4 | 5 | Inv. |
| 29 | 4-6 | 2.34 | 5 | 5 | 4 | 5 | Inv. |
| 30 | 4-13 | 2.35 | 5 | 5 | 5 | 5 | Inv. |
| 31 | 4-19 | 2.33 | 5 | 5 | 4 | 5 | Inv. |

As apparent from Table 3, it was shown that sample No. 19 containing comparative couplers C-1 and C-2 was markedly insufficient for color reproduction. It was also shown that sample Nos. 20 and 21, in which comparative coupler "b" or "d" was used in an amount of 50 mol% of the sum of C-1 and C-2, and silver halide was changed to 70 mol%, were greatly improved in blue color reproduction but were insufficient for color reproduction of cyan and green, and the maximum density was low, leading to insufficient blackness. On the contrary, it was proved that samples containing the coupler of formula (2), (3) or (4) (in which the coupler was used in an amount of 50 mol% of the sum of C-1 and C-2, and silver halide was changed to 70 mol%) exhibited superior color reproduction, enhanced maximum density and improved blackness.

## Claims

1. A silver halide colour photographic material comprising a support having thereon at least one silver halide emulsion layer comprising a coupler represented by formula (2), (3) or (4): 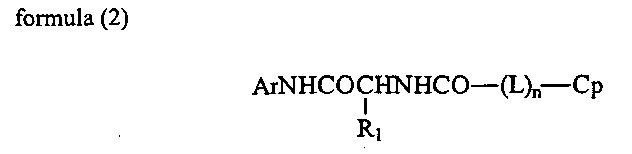 wherein Ar is an aryl group or a heterocyclic group; R₁ is an alkyl group, an aryl group or a heterocyclic group; L is a bivalent linkage group; n is 0 or 1; and Cp is a pyrazolotriazole coupler residue; 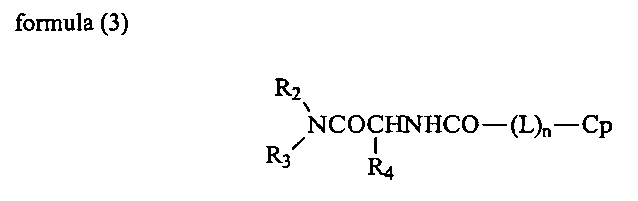 wherein R₂, R₃, and R₄ are each an alkyl group, an aryl group or a heterocyclic group, or R₂ and R₃ combine with each other to form a ring; L is a bivalent linkage group; n is 0 or 1; and Cp is a pyrazolotriazole coupler residue; 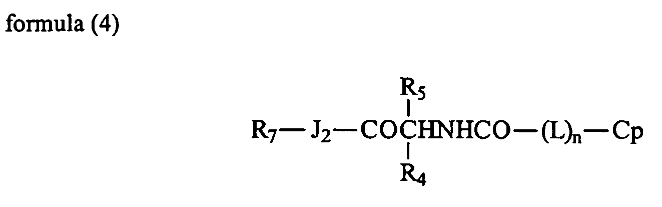 wherein R₅ is an unsubstituted alkyl group having 5 or more carbon atoms; R₆ is a hydrogen atom, an alkyl group, an aryl group or a heterocyclic group; R₇ is an alkyl group, an aryl group or a heterocyclic group; J₂ is -O- or -NR₁₁-, in which R₁₁ is a hydrogen atom, an alkyl group, an aryl group or a heterocyclic group; L is a bivalent linkage group; n is 0 or 1; Cp is a pyrazolotriazole coupler residue.

2. A photographic material according to claim 1, wherein the coupler residue of formulas (2), (3) and (4) is represented by the following formula (6): formula (6) 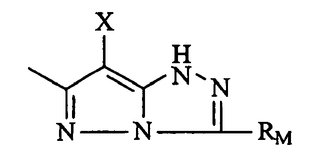 wherein X is a hydrogen atom, a halogen atom or a group capable of being released upon coupling with an oxidation product of a colour developing agent; and R_{M} is a univalent substituent group.

3. A photographic material according to claim 1, wherein in formula (2), R₁ is an alkyl group or an aryl group.

4. A photographic material according to claim 3, wherein said R₁ is an alkyl group.

5. A photographic material according to claim 1, wherein in formula (3), R₂, R₃ and R₄ are each an alkyl group or an aryl group.

6. A photographic material according to claim 1, wherein in formula (3), R₄ is an alkyl group.

7. A photographic material according to claim 5, wherein R₄ is an alkyl group.

8. A photographic material according to claim 1, wherein in formula (4), R₆ is a hydrogen atom or an alkyl group.

9. A photographic material according to claim 8, wherein R⁶ is a hydrogen atom.

10. A photographic material according to claim 1, wherein in formula (4), J₂ is -NR₁₁-.

11. A photographic material according to claim 1, wherein the silver halide emulsion layer comprises the coupler in an amount of from 1x10⁻³ to 1 mol per mol of silver halide.

12. A colour photographic material according to claim 1, comprising at least one red-sensitive silver halide emulsion layer comprising a coupler represented by formula (2), (3) or (4) as defined in claim 1.

13. A colour photographic material according to claim 1, comprising at least one red-sensitive silver halide emulsion layer, at least one green-sensitive silver halide emulsion layer and at least one blue-sensitive silver halide emulsion layer, wherein the red-sensitive silver halide emulsion comprises a coupler represented by formula (2), (3) or (4) as defined in claim 1.

## Patentansprüche

1. Farbphotographisches Silberhalogenid-Aufzeichnungsmaterial, umfassend einen Schichtträger mit mindestens einer darauf befindlichen Silberhalogenidemulsionsschicht, die einen Kuppler der Formel (2), (3) oder (4) umfasst: 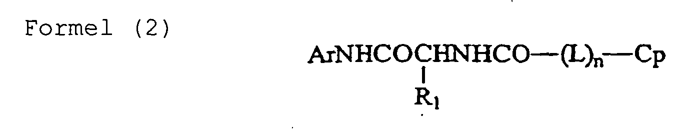 worin Ar für eine Arylgruppe oder eine heterocyclische Gruppe steht; R₁ für eine Alkylgruppe, eine Arylgruppe oder eine heterocyclische Gruppe steht; L für eine zweiwertige verbindende Gruppe steht; n 0 oder 1 ist; und Cp ein Pyrazolotriazolkupplerrest ist; 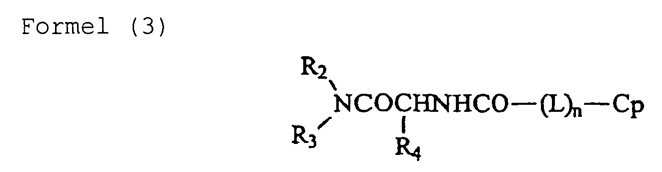 worin R₂, R₃ und R₄ jeweils für eine Alkylgruppe, eine Arylgruppe oder eine heterocyclische Gruppe stehen oder R₂ und R₃ miteinander unter Bildung eines Rings verbunden sind; L für eine zweiwertige verbindende Gruppe steht; n 0 oder 1 ist und Cp ein Pyrazolotriazolkupplerrest ist; 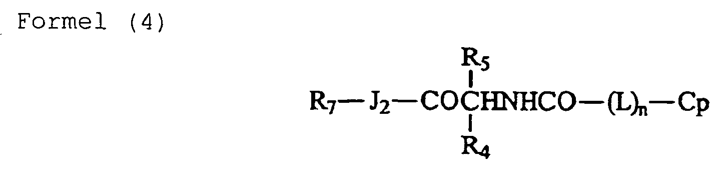 worin R₅ für eine unsubstituierte Alkylgruppe mit 5 oder mehr Kohlenstoffatomen steht; R₆ für ein Wasserstoffatom, eine Alkylgruppe, eine Arylgruppe oder eine heterocyclische Gruppe steht; R₇ für eine Alkylgruppe, eine Arylgruppe oder eine heterocyclische Gruppe steht; J₂ für -O- oder -NR₁₁- steht, worin R₁₁ ein Wasserstoffatom, eine Alkylgruppe, eine Arylgruppe oder eine heterocyclische Gruppe ist; L für eine zweiwertige verbindende Gruppe steht; n 0 oder 1 ist, Cp ein Pyrazolotriazolkupplerrest ist.

2. Photographisches Aufzeichnungsmaterial nach Anspruch 1, wobei der Kupplerrest der Formeln (2), (3) und (4) durch die folgende Formel (6) dargestellt wird: 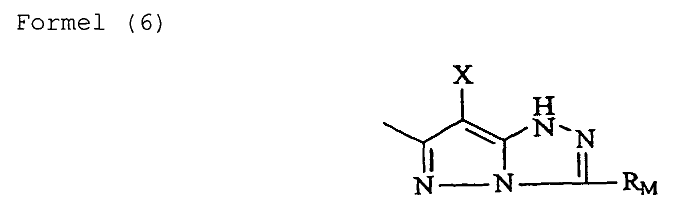 worin X für ein Wasserstoffatom, ein Halogenatom oder eine Gruppe, die bei Kopplung mit einem Oxidationsprodukt einer Farbentwicklersubstanz freisetzbar ist, steht; und R_{M} für eine einwertige Substituentengruppe steht.

3. Photographisches Aufzeichnungsmaterial nach Anspruch 1, wobei in der Formel (2) R₁ für eine Alkylgruppe oder eine Arylgruppe steht.

4. Photographisches Aufzeichnungsmaterial nach Anspruch 3, wobei das R₁ eine Alkylgruppe ist.

5. Photographisches Aufzeichnungsmaterial nach Anspruch 1, wobei in der Formel (3) R₂, R₃ und R₄ jeweils für eine Alkylgruppe oder eine Arylgruppe stehen.

6. Photographisches Aufzeichnungsmaterial nach Anspruch 1, wobei in der Formel (3) R₄ eine Alkylgruppe ist.

7. Photographisches Aufzeichnungsmaterial nach Anspruch 5, wobei R₄ eine Alkylgruppe ist.

8. Photographisches Aufzeichnungsmaterial nach Anspruch 1, wobei in der Formel (4) R₆ für ein Wasserstoffatom oder eine Alkylgruppe steht.

9. Photographisches Aufzeichnungsmaterial nach Anspruch 8, wobei R⁶ ein Wasserstoffatom ist.

10. Photographisches Aufzeichnungsmaterial nach Anspruch 1, wobei in der Formel (4) J₂ -NR₁₁- ist.

11. Photographisches Aufzeichnungsmaterial nach Anspruch 1, wobei die Silberhalogenidemulsionsschicht den Kuppler in einer Menge von 1 x 10⁻³ bis 1 Mol pro Mol Silberhalogenid umfasst.

12. Farbphotographisches Aufzeichnungsmaterial nach Anspruch 1, umfassend mindestens eine rotempfindliche Silberhalogenidemulsionsschicht, die einen Kuppler der Formel (2), (3) oder (4) gemäß der Definition in Anspruch 1 umfasst.

13. Farbphotographisches Aufzeichnungsmaterial nach Anspruch 1, das mindestens eine rotempfindliche Silberhalogenidemulsionsschicht, mindestens eine grünempfindliche Silberhalogenidemulsionsschicht und mindestens eine blauempfindliche Silberhalogenidemulsionsschicht umfasst, wobei die rotempfindliche Silberhalogenidemulsionsschicht einen Kuppler der Formel (2), (3) oder (4) gemäß der Definition in Anspruch 1 umfasst.

## Revendications

1. Matériau photographique couleur à l'halogénure d'argent, comprenant un support sur lequel se trouve au moins une couche d'émulsion d'halogénure d'argent comprenant un agent de couplage représenté par la formule (2), (3) ou (4) : 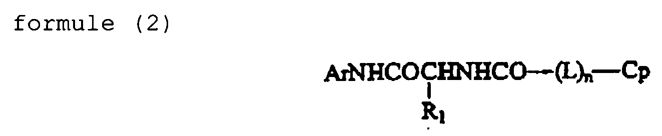 dans laquelle Ar est un groupe aryle ou un groupe hétérocyclique ; R₁ est un groupe alkyle, un groupe aryle ou un groupe hétérocyclique ; L est un groupe de liaison bivalent ; n est 0 ou 1 ; et Cp est un résidu d'agent de couplage pyrazolotriazole ; 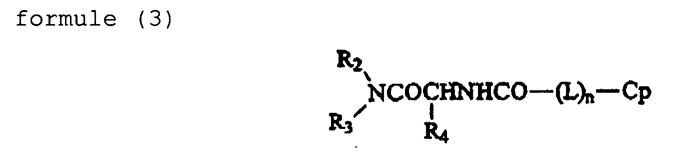 dans laquelle R₂, R₃ et R₄ sont chacun un groupe alkyle, un groupe aryle ou un groupe hétérocyclique, ou R₂ et R₃ se combinent entre eux pour former un cycle ; L est un groupe de liaison bivalent ; n est 0 ou 1 ; et Cp est un résidu d'agent de couplage pyrazolotriazole ; 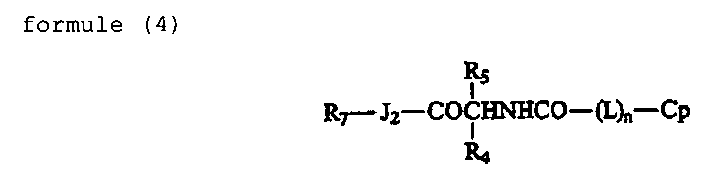 dans laquelle R₅ est un groupe alkyle non substitué ayant 5 atomes de carbone ou plus ; R₆ est un atome d'hydrogène, un groupe alkyle, un groupe aryle ou un groupe hétérocyclique ; R₇ est un groupe alkyle, un groupe aryle ou un groupe hétérocyclique ; J₂ est -O- ou -NR₁₁-, où R₁₁ est un atome d'hydrogène, un groupe alkyle, un groupe aryle ou un groupe hétérocyclique ; L est un groupe de liaison bivalent ; n est 0 ou 1 ; Cp est un résidu d'agent de couplage pyrazolotriazole.

2. Matériau photographique selon la revendication 1, dans lequel le résidu d'agent de couplage des formules (2), (3) et (4) est représenté par la formule (6) suivante : 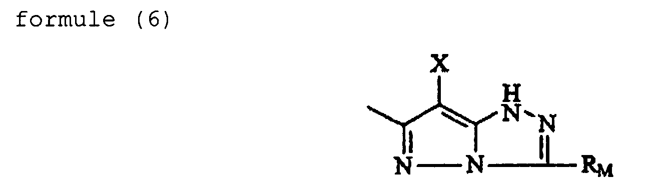 dans laquelle X est un atome d'hydrogène, un atome d'halogène ou un groupe apte à être libéré par couplage avec un produit d'oxydation d'un agent développateur de couleur ; et R_{M} est un groupe substituant univalent.

3. Matériau photographique selon la revendication 1, dans lequel, dans la formule (2), R₁ est un groupe alkyle ou un groupe aryle.

4. Matériau photographique selon la revendication 3, dans lequel ledit R₁ est un groupe alkyle.

5. Matériau photographique selon la revendication 1, dans lequel, dans la formule (3), R₂, R₃ et R₄ sont chacun un groupe alkyle ou un groupe aryle.

6. Matériau photographique selon la revendication 1, dans lequel, dans la formule (3), R₄ est un groupe alkyle.

7. Matériau photographique selon la revendication 5, dans lequel R₄ est un groupe alkyle.

8. Matériau photographique selon la revendication 1, dans lequel, dans la formule (4), R₆ est un atome d'hydrogène ou un groupe alkyle.

9. Matériau photographique selon la revendication 8, dans lequel R₆ est un atome d'hydrogène.

10. Matériau photographique selon la revendication 1, dans lequel, dans la formule (4), J₂ est -NR₁₁-.

11. Matériau photographique selon la revendication 1, dans lequel la couche d'émulsion d'halogénure d'argent comprend l'agent de couplage en une quantité de 1x10⁻³ à 1 mole par mole d'halogénure d'argent.

12. Matériau photographique couleur selon la revendication 1, comprenant au moins une couche d'émulsion d'halogénure d'argent sensible au rouge comprenant un agent de couplage représenté par la formule (2), (3) ou (4) telle que définie dans la revendication 1.

13. Matériau photographique couleur selon la revendication 1, comprenant au moins une couche d'émulsion d'halogénure d'argent sensible au rouge, au moins une couche d'émulsion d'halogénure d'argent sensible au vert et au moins une couche d'émulsion d'halogénure d'argent sensible au bleu, dans lequel l'émulsion d'halogénure d'argent sensible au rouge comprend un agent de couplage représenté par la formule (2), (3) ou (4) telle que définie dans la revendication 1.
